(19) Europäisches Patentamt
European Patent Office
Office européen des brevets

(11) EP 1 621 540 A1

(12) # EUROPEAN PATENT APPLICATION
published in accordance with Art. 158(3) EPC

(43) Date of publication:
**01.02.2006 Bulletin 2006/05**

(21) Application number: **04728443.5**

(22) Date of filing: **20.04.2004**

(51) Int Cl.:
***C07D 471/10*** *(1985.01)*
***A61P 11/06*** *(2000.01)*
***A61P 1/16*** *(2000.01)*
***A61P 11/02*** *(2000.01)*
***A61P 1/04*** *(2000.01)*
***A61P 17/06*** *(2000.01)*
***A61P 37/08*** *(2000.01)*
***A61P 11/00*** *(2000.01)*
***A61P 3/10*** *(2000.01)*
***A61K 31/499*** *(2000.01)*
***A61P 13/12*** *(2000.01)*
***A61P 19/02*** *(2000.01)*
***A61P 27/02*** *(2000.01)*
***A61P 37/02*** *(2000.01)*
***A61P 31/18*** *(2000.01)*
***A61P 9/10*** *(2000.01)*
***A61P 35/04*** *(2000.01)*

(86) International application number:
**PCT/JP2004/005610**

(87) International publication number:
**WO 2004/094424 (04.11.2004 Gazette 2004/45)**

(84) Designated Contracting States:
**AT BE BG CH CY CZ DE DK EE ES FI FR GB GR HU IE IT LI LU MC NL PL PT RO SE SI SK TR**

(30) Priority: **21.04.2003 JP 2003116235**

(71) Applicant: **ONO PHARMACEUTICAL CO., LTD.
Osaka-shi,
Osaka 541-8526 (JP)**

(72) Inventors:
- **Nishizawa, Rena,
c/o Ono Pharmaceutical Co. Ltd
Mishima-gun,
Osaka 618-8585 (JP)**
- **Takaoka, Yoshikazu,
c/o Ono Pharmaceutical Co. Ltd
Mishima-gun,
Osaka 618-8585 (JP)**
- **Shibayama, Shiro,
c/o Ono Pharmaceutical Co. Ltd
Mishima-gun,
Osaka 618-8585 (JP)**

(74) Representative: **Henkel, Feiler & Hänzel
Möhlstrasse 37
81675 München (DE)**

(54) **NITROGEN-CONTAINING HETEROCYCLIC COMPOUNDS AND USE THEREOF**

(57) A pharmaceutical composition comprising, as an active ingredient, the compounds represented by formula (1)

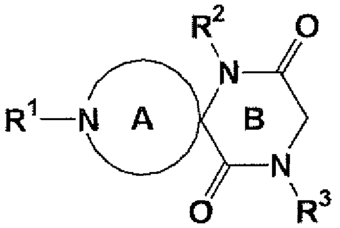

(I)

(wherein all of the symbols have the same meanings as defined in specification.), salts thereof, solvates thereof, or prodrugs thereof. The compounds represented by formula (I) are used for prevention and/or treatment of various inflammatory diseases (asthma, nephritis, nephropathy, hepatitis, arthritis, chronic rheumatoid arthritis, rhinitis, conjunctivitis, ulcerative colitis and the like), immunologic diseases (treatment of autoimmune diseases, transplant rejection, immunosuppression, psoriasis, multiple sclerosis and the like), human immunodeficiency virus infection (acquired immunodeficiency syndrome and the like), allergic diseases (atopic dermatitis, nettle rash, allergic bronchopulmonary aspergillosis, allergic eosinophilic gastroenteritis and the like), suppression of ischemia-reperfusion injury, acute respiratory distress syndrome, shock accompanied by bacterial infection, diabetes mellitus, or metastasis and the like.

EP 1 621 540 A1

**Description**

TECHNICAL FIELD

The present invention relates to

(1) a compound represented by formula (I)

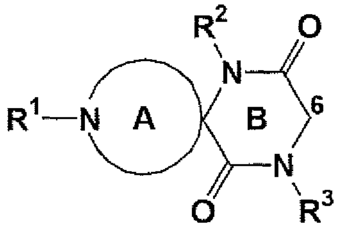

(I)

(wherein all symbols have the same meanings as defined hereinafter.), a salt thereof, a solvate thereof or a prodrug thereof, and
(2) a pharmaceutical composition comprising, as an active ingredient, the compound represented by formula (I), a salt thereof, a solvate thereof or a prodrug thereof.

BACKGROUND ART

Chemokine is known as a basic protein having endogeneous leukocyte chemotactic and activating abilities and strong heparin-binding abilities. At present, it is considered that chemokine is related to not only the control of infiltration of specific leukocyte at the time of inflammations and immune responses but also the development and homing of lymphocyte under physiological conditions and migration of hemocyte precursor cells and somatic cells.
Differentiation, proliferation and cell death of hemocytes are controlled by various types of cytokine. In the living body, inflammations are found topically and differentiation, maturation and the like of lymphocytes are carried out at certain specified sites. That is, various necessary cells migrate into certain specified sites and accumulate therein to cause a series of inflammations and immune responses. Accordingly, migration of cells is also an indispensable phenomenon in addition to differentiation, proliferation and death of cells.
Migration of hemocytes in the living body starts firstly in the development stage by the shift of hematopoiesis started in the AGM (aorta gonad mesonephros) region into permanent hematopoiesis in bone marrow via fetal liver. Furthermore, precursor cells of T cells and thymus dendritic cells migrate from the fetal liver into the bone marrow and then into the thymus gland and cytodifferentiate under thymus environment. The T cell which received clone selection migrates into secondary lymphoid tissues and takes part in an immune response in the periphery. The Langerhans' cell of the skin activated and differentiated by capturing an antigen migrates into the T cell region of a topical lymph node and activates naive T cell therein as a dendritic cell. The memory T cell performs its homing again into the lymph node via lymphatic and blood vessels. Also, B cell, T cell in the intestinal epithelium, $\gamma\delta$ T cell, NKT cell and dendritic cell migrate from bone marrow without passing through the thymus gland and differentiate to take part in an immune response.
Chemokine is deeply related to the migration of these various cells. For example, MTP3β (macrophage inflammatory protein 3β), SLC (secondary lymphoid tissue chemokine) and its receptor CCR7 play an important role in the migration and homing of naive T cell, memory T cell and the mature dendritic cell which captured an antigen into a topical lymphoid tissue for the dendritic cells to encounter efficiently with the T cells. The T cell and dendritic cell necessary for controlling antigen-specific immune responses are hardly observed in the secondary lymph node of a PLT mouse having deficiency in the expression of SLC *(J. Exp. Med.,* 189(3), 451 (1999)).
MDC (macrophage - derived chemokine), TARC (thymus and activation - regulated chemokine) and its receptor CCR4 play an important role in the migration of Th2 cell into topical sites in immune and inflammatory responses in which the Th2 cell is related. In a rat fulminant hepatitis model (P. acnes + LPS), an anti-TARC antibody suppressed increase of the amount of ALT in blood and increase of the expressing amounts of TNFα and FasL in the liver and also improved lethality of the rats *(J. Clin. Invest.,* 102, 1933 (1998)). Also, an anti-MDC antibody decreased the number of eosinophils accumulated in the lung interstitium and suppressed airway hypersensitivity in a mouse OVA-induced airway hypersensitivity model (J. *Immunology,* 163, 403 (1999)).
MCP-1 (monocyte chemoattractant protein-1) and its receptor CCR2 are related to the infiltration of macrophage into inflammation sites. An anti-MCP-1 antibody showed an effect to suppress infiltration of monocyte and macrophage

into glomerulus in a rat anti-Thy1.1 antibody glomerular nephritis model *(Kidney Int.,* 51, 770 (1997)).

Thus, chemokine receptors are greatly related to the control of inflammation and immune responses through a mechanism in which they are expressed at certain specified periods in variously specific cells and the effector cells are accumulated in a region where chemokine is produced.

Acquired immunodeficiency syndrome (called AIDS) which is induced by human immunodeficiency virus (hereinafter referred to as "HIV") is one of the diseases of which their therapeutic methods are most earnestly desired in recent years. Once infection with HIV is completed in a CD4-positive cell which is a principal target cell, HIV repeats its proliferation in the body of the patient and, sooner or later, completely destroys T cell which takes charge of the immunological function. During this process, the immunological function is gradually reduced to cause fever, diarrhea, lymph node enlargement and the like various immunodeficiency conditions which are apt to cause complications with pneumocystis carinii pneumonia and the like various opportunistic infections. Such conditions are the onset of AIDS, and it is well known that they induce and worsen Kaposi sarcoma and the like malignant tumors.

As the recent preventive and therapeutic methods for AIDS, attempts have been made to, e.g., (1) inhibit growth of HIV by the administration of a reverse transcriptase inhibitor or a protease inhibitor and (2) prevent or alleviate opportunistic infections by the administration of a drug having immunopotentiation activity.

Helper T cells which take charge of the central of immune system are mainly infected with HIV. It is known since 1985 that HIV uses the membrane protein CD4 expressing on the membrane of T cells in the infection *(Cell,* 52, 631 (1985)). The CD4 molecule is composed of 433 amino acid residues, and its expression can be found in macrophages, some B cells, vascular endothelial cells, Langerhans' cells in skin tissues, dendritic cells in lymphoid tissues, glia cells of the central nervous system and the like, in addition to the mature helper T cells. However, since it has been revealed that the infection with HIV is not completed by the CD4 molecule alone, a possibility has been suggested on the presence of factors other than the CD4 molecule, which are related to the infection of cells with HIV.

In 1996, a cell membrane protein called Fusin was identified as a factor other than the CD4 molecule, which is related to the HIV infection *(Science,* 272, 872 (1996)). It was confirmed that this Fusin molecule is a receptor (namely, CXCR4) of stromal derived factor-1 (hereinafter referred to as "SDF-1"). In addition, it was confirmed also *in vitro* that the SDF-1 specifically inhibits infection of T cell tropic (X4) HIV *(Nature,* 382, 829 (1996), *Nature,* 382, 833 (1996)). That is, it is considered that the HIV infection was inhibited by the binding of SDF-1 to CXCR4 preceding HIV, thereby depriving HIV of a foothold for infecting cells.

Also at that time, it was discovered that another chemokine receptor CCR5, which is a receptor of RANTES, MIP-1$\alpha$ and MIP-1$\beta$, is also used at the time of the infection with a macrophage tropic (R5) HIV *(Science,* 272, 1955 (1996)).

Accordingly, substances which can compete with CXCR4 and CCR5 for HIV, or which can bind to HIV virus thus causing the virus unable to bind to CXCR4 and CCR5, could become HIV infection inhibitors. Also, there is a case in which a low molecular compound initially discovered as an HIV infection inhibitor was actually a CXCR4 antagonist *(Nature Medicine,* 4, 72 (1998)).

Furthermore, substances which can compete with CCR5 for HIV, or which can bind to HIV virus thus causing the virus unable to bind to CCR5, could become HIV infection inhibitors.

Based on the above, it is considered that the chemokine receptors are deeply related to various inflammatory diseases, immunologic diseases such as autoimmune diseases or allergic diseases, or HIV infection. For example, it is considered that they are related to asthma, nephritis, nephropathy, hepatitis, arthritis, chronic rheumatoid arthritis, rhinitis, conjunctivitis, ulcerative colitis, transplant rejection, immunosuppression, psoriasis, multiple sclerosis, human immunodeficiency virus (acquired immunodeficiency syndrome and the like), atopic dermatitis, nettle rash, allergic bronchopulmonary aspergillosis, allergic eosinophilic gastroenteritis, ischemia-reperfusion injury, acute respiratory distress syndrome, shock accompanied by bacterial infection, diabetes mellitus, metastasis and the like.

It is described that the compounds of formula (Z)

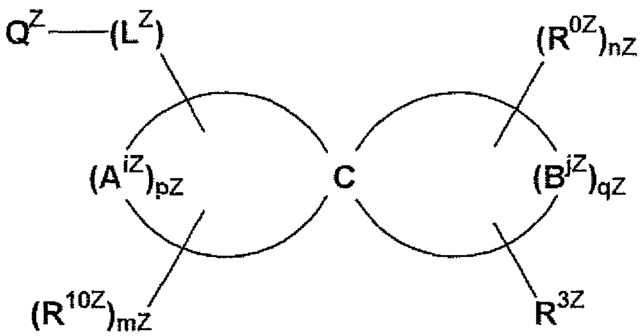

(Z)

(wherein the atoms $A^{iZ}$ and $B^{jZ}$ are independently selected from carbon, nitrogen, oxygen and sulfur, provided that at least one atom of $A^{iZ}$ is carbon, and at least one atom $B^{jZ}$ is carbon.); the rings of the spirobicycle formed by $A^{iZ}$ and $B^{iZ}$, respectively, may optionally be partly unsaturated, pZ and qZ are independently numbers from 2 to 6, mZ is a number from 0 to pZ, $R^{10Z}$ is the same or different and is a non-interfering substituent independently selected from hydrogen, alkyl, halosubstituted alkyl, alkenyl, alkynyl, cycloalkyl, =O and =S *etc.,* nZ is a number from 0 to qZ, $R^{0Z}$ is the same or different and is a non-interfering substituent independently selected from hydrogen, alkyl, halosubstituted alkyl, alkenyl, alkynyl, cycloalkyl, =O, and =S *etc.,* the linking group -($L^z$)- is a single bond or a divalent substituted or unsubstituted chain of 1 to 10 atoms selected from the group consisting of carbon, nitrogen, sulfur, and oxygen, $Q^Z$ is a basic group containing one or more basic radical(s), and $R^{3Z}$ is an acidic group containing one or more acid radical(s)) are useful for inhibiting platelet aggregation (ref. the specification of WO97/11940).

Furthermore, it is described that the compounds of formula (Y)

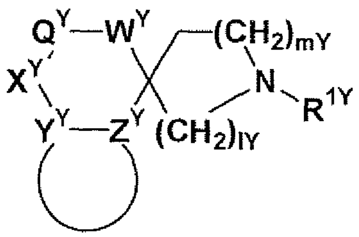
(Y)

(wherein, mY or 1Y are each independently 0, 1, 2, 3, 4 or 5, $R^{1Y}$ is hydrogen, C1-8 alkyl, C2-8 alkenyl, C2-8 alkynyl *etc.,* $W^Y$ is a single bond, C1-3 alkyl or C1-3 alkyl substituted with oxo *etc.*, $Q^Y$ is $-NR^2-$, -O-, -S-, -S(O)- or $-SO_2-$, $X^Y$ is a single bond, C1-3 alkyl or C1-3 alkyl substituted with oxo *etc.,* $Y^Y$-$Z^Y$ ring is phenyl, naphthyl or hetero aryl, and wherein the definition of each symbol is an excerpt partially.) are useful as modulators of the chemokine receptors (ref. the specification of WO98/25605)

On the other hand, it is reported that triazaspiro[5.5]undecane derivatives, quaternary ammonium salts thereof or N-oxides thereof, or salts thereof regulate the effect of chemokine/chemokine receptor (CCR), so that they are used for prevention and/or treatment of various inflammatory diseases, asthma, atopic dermatitis, urticaria, allergic diseases (allergic bronchopulmonary aspergillosis or allergic eosinophilic gastroenteritis etc.), nephritis, nephropathy, hepatitis, arthritis, rheumatoid arthritis, psoriasis, rhinitis, conjunctivitis, ischemic reperfusion disorder, multiple sclerosis, ulcerative colitis, acute respiratory distress syndrome, cytotoxic shock, diabetes, autoimmune disease, transplanted organ rejection reactions, immunosuppression, cancer metastasis and acquired immune deficiency syndrome (ref. the specification of WO01/40227).

DISCLOSURE OF THE INVENTION

An object of the present invention is to provide a compound having chemokine antagonistic activity against a chemokine receptor, which is useful as a preventive and/or therapeutic agent for human immunodeficiency virus infection etc.

The present inventors have made investigations so as to find a compound having antagonistic activity against a chemokine. Consequently, the inventors have found that the compound represented by formula (I) of the present invention can meet the object, and the present invention has been accomplished. Particularly, the compound of the present invention is suitable as a CCR5 receptor antagonist.

Specifically, the present invention relates to:

1. A compound represented by formula (I):

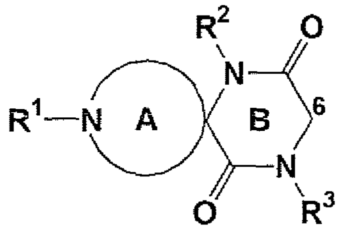

(I)

wherein ring A represents a 3- to 15-membered nitrogen-containing mono-, bi- or tri-cyclic hetero ring which may have a substituent(s);
ring B may have, at the 6-position, an aliphatic hydrocarbon group which may have a substituent(s), a cyclic group which may have a substituent(s), a hydroxyl group which may be protected, a carboxyl group which may be protected, or a carbamoyl group which may be substituted, or

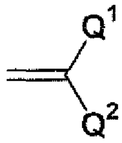

wherein $Q^1$ and $Q^2$ each independently represents hydrogen, an aliphatic hydrocarbon group which may have a substituent(s), a cyclic group which may have a substituent(s), a hydroxyl group which may be protected, a carboxyl group which may be protected, or a carbamoyl group which may be substituted;
$R^1$ represents hydrogen, an aliphatic hydrocarbon group which may have a substituent(s), or a cyclic group which may has a substituent(s);
$R^2$ and $R^3$ each independently represents hydrogen, an aliphatic hydrocarbon group which may have a substituent(s), a cyclic group which may have a substituent(s), a hydroxyl group which may be protected, a carboxyl group which may be protected, or a carbamoyl group which may be substituted, and
wherein, when ring A is

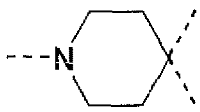 ,

ring A has a substituent(s) other than $R^1$, or
a salt thereof, a solvate thereof, or a prodrug thereof,
2. the compound according to the above 1, wherein ring A is a 4- to 8-membered nitrogen-containing mono-cyclic hetero ring or a 9- to 15-membered nitrogen-containing bi- or tri-cyclic hetero ring which may have a substituent(s), or a salt thereof, a solvate thereof, or a prodrug thereof,
3. the compound according to the above 1, wherein ring A is

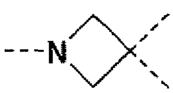 , 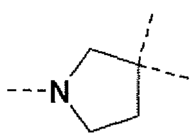 , 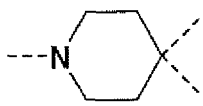 , 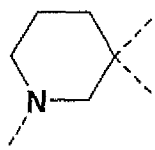 ,

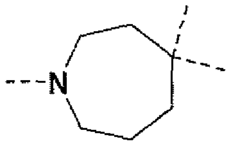 , 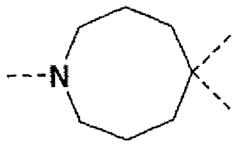 , 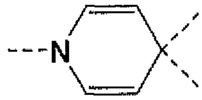 , 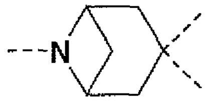 ,

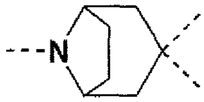 , 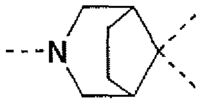 , 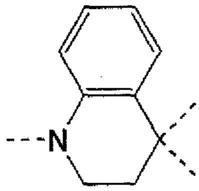 , or 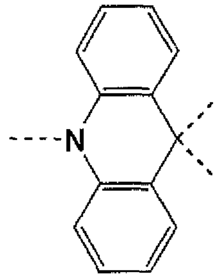

and wherein, when ring A is

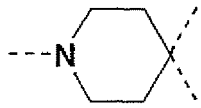 ,

ring A has a substituent(s) other than $R^1$, or a salt thereof, a solvate thereof, or a prodrug thereof,

4. the compound according to the above 1, wherein $R^1$ is an aliphatic hydrocarbon group which may have a substituent(s), a salt thereof, a solvate thereof, or a prodrug thereof,

5. the compound according to the above 1, wherein ring B is an aliphatic hydrocarbon group which may have a substituent(s), a salt thereof, a solvate thereof, or a prodrug thereof,

6. the compound according to the above 1, wherein $R^3$ is hydrogen, a salt thereof, a solvate thereof, or a prodrug thereof,

7. a pharmaceutical composition comprising, as an active ingredient, the compound according to the above 1, a salt thereof, a solvate thereof, or a prodrug thereof,

8. the pharmaceutical composition according to the above 7, which is a chemokine receptor antagonist,

9. the pharmaceutical composition according to the above 8, wherein the chemokine receptor is CCR5,

10. the pharmaceutical composition according to the above 7, which is a preventive and/or therapeutic agent for CCR5-related diseases,

11. the pharmaceutical composition according to the above 7, which is a preventive and/or therapeutic agent for human immunodeficiency virus infection,

12. the pharmaceutical composition according to the above 7, which is a preventive and/or therapeutic agent for acquired immunodeficiency syndrome,

13. the pharmaceutical composition according to the above 7, which is a preventive and/or therapeutic agent for transplanted organ rejection reactions,

14. a medicament which comprises a combination of the compound represented by formula (I) according to the above 1, a salt thereof, a solvate thereof, or a prodrug thereof with one or at least two of agents selected from reverse transcriptase inhibitors, protease inhibitors, CCR2 antagonists, CCR3 antagonists, CCR4 antagonists, CXCR4 antagonists, fusion inhibitors, HIV integrase inhibitors, antibodies against a surface antigen of HIV-1 and vaccines against HIV-1,

15. a method for preventing and/or treating CCR5-related diseases in a mammal, which comprises administering to a mammal an effective amount of the compound represented by formula (I) according to the above 1, a salt thereof, a solvate thereof, or a prodrug thereof,

16. a method for preventing and/or treating immunodeficiency virus infection in a mammal, which comprises administering to a mammal an effective amount of the compound represented by formula (I) according to the above 1, a salt thereof, a solvate thereof, or a prodrug thereof,

17. use of the compound represented by formula (I) according to the above 1, a salt thereof, a solvate thereof, or a prodrug thereof for the manufacture of a preventive and/or therapeutic agent for CCR5-related diseases, and

18. use of the compound represented by formula (I) according to the above 1, a salt thereof, a solvate thereof, or

a prodrug thereof for the manufacture of a preventive and/or therapeutic agent for human immunodeficiency virus infection.

In the specification, the "aliphatic hydrocarbon group" in the "aliphatic hydrocarbon group which may have a substituent(s)" includes "linear or branched chain C1-18 hydrocarbon group". The "linear or branched chain C1-18 hydrocarbon group" includes C1-18 alkyl, C2-18 alkenyl, C2-18 alkynyl and the like. In this case, C1-18 alkyl includes methyl, ethyl, propyl, isopropyl, butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, octadecyl and isomer groups thereof and the like. The C2-18 alkenyl includes vinyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, butadienyl, pentadienyl, hexadienyl, heptadienyl, octadienyl, nonadienyl, decadienyl, undecadienyl, dodecadienyl, tridecadienyl, tetradecadienyl, pentadecadienyl, hexadecadienyl, heptadecadienyl, octadecadienyl, hexatrienyl, heptatrienyl, octatrienyl, nonatrienyl, decatrienyl, undecatrienyl, dodecatrienyl, tridecatrienyl, tetradecatrienyl, pentadecatrienyl, hexadecatrienyl, heptadecatrienyl, octadecatrienyl and isomer groups thereof and the like. The C2-18 alkynyl includes ethynyl, propynyl, butynyl, pentynyl, hexynyl, heptynyl, octynyl, nonynyl, decynyl, undecynyl, dodecynyl, tridecynyl, tetradecynyl, pentadecynyl, hexadecynyl, heptadecynyl, octadecynyl, butadiynyl, pentadiynyl, hexadiynyl, heptadiynyl, octadiynyl, nonadiynyl, decadiynyl, undecadiynyl, dodecadiynyl, tridecadiynyl, tetradecadiynyl, pentadecadiynyl, hexadecadiynyl, heptadecadiynyl, octadecadiynyl, hexatriynyl, heptatriynyl, octatriynyl, nonatriynyl, decatriynyl, undecatriynyl, dodecatriynyl, tridecatriynyl, tetradecatriynyl, pentadecatriynyl, hexadecatriynyl, heptadecatriynyl, octadecatriynyl and isomer groups thereof and the like.

As the "aliphatic hydrocarbon group" in the "aliphatic hydrocarbon group which may have a substituent(s)" represented by $R^1$, a C1-10 aliphatic hydrocarbon group is preferred, C1-6 alkyl and C2-6 alkenyl are more preferred, and C1-6 alkyl is particularly preferred. In particular, methyl and ethyl are preferred.

In the specification, the "substituent" in the "aliphatic hydrocarbon group which may have a substituent(s)" represented by $R^1$ includes a substituent selected from the following first group, a substituent selected from the following second group and a cyclic group which may have a substituent(s), and the like, and 1 to 5 of these substituents may be substituted at substitutable positions.

<First group>

(1) halogen (e.g., chlorine, bromine, fluorine, iodine), (2) nitro, (3) trifluoromethyl, (4) trifluoromethoxy, (5) cyano, (6) oxo,

<Second group>

(1) $-SR^a$, (2) $-SO_2R^a$, (3) $-SO_2NR^bR^{b'}$, (4) $-S(O)R^a$, (5) $-OR^a$, (6) $-OCOR^a$, (7) $-NR^aSO_2R^{a'}$, (8) $-NR^bR^{b'}$, (9) $-NR^aCOR^{a'}$, (10) $-NR^aCOOR^{a'}$ (11) $-NR^aCONR^bR^{b'}$, (12) $-N(SO_2R^a)_2$, (13) $-COR^a$, (14) $-COOR^a$, (15) $-CONR^bR^{b'}$, (16) $-CONR^aCOR^{a'}$, (17) $-COCOOR^a$, (18) $B(OR^a)_2$,

wherein $R^a$, $R^{a'}$, $R^b$ and $R^{b'}$ each independently represents hydrogen, a cyclic group (ring 1) which may have a substituent(s) or an aliphatic hydrocarbon group which may have a substituent(s), or, $R^b$ and $R^{b'}$ may be taken together with the adjacent nitrogen to represent (1) -C2-6 alkylene (e.g., methylene, ethylene, trimethylene, tetramethylene, pentamethylene, hexamethylene and isomer groups thereof and the like)-, (2) -(C2-6 alkylene)-O-(C2-6 alkylene)-, (3) -(C2-6 alkylene)-S-(C2-6 alkylene)-, (4) -(C2-6 alkylene)-$NR^{N1}$-(C2-6 alkylene)- (wherein $R^{N1}$ represents hydrogen, a cyclic group (ring 1) which may have a substituent(s) or C1-8 alkyl may be substituted with the "cyclic group (ring 1) which may have a substituent(s)").

The "aliphatic hydrocarbon group" in the "aliphatic hydrocarbon group which may have a substituent(s)" represented by $R^a$, $R^{a'}$, $R^b$ and $R^{b'}$ includes a "linear or branched chain C1-8 hydrocarbon group" and the like. The "linear or branched chain C1-8 hydrocarbon group" includes C1-8 alkyl, C2-8 alkenyl, C2-8 alkynyl and the like. In this case, the C1-8 alkyl includes methyl, ethyl, propyl, isopropyl, butyl, sec-butyl, tert-butyl, pentyl, hexyl, heptyl, octyl and isomer groups thereof and the like. The C2-8 alkenyl includes vinyl, propenyl, butenyl, pentenyl, hexenyl, heptenyl, octenyl, butadienyl, pentadienyl, hexadienyl, heptadienyl, octadienyl, hexatrienyl, heptatrienyl, octatrienyl and isomer groups thereof and the like. As the C2-8 alkynyl, for example, ethynyl, propynyl, butynyl, pentynyl, hexynyl, heptynyl, octynyl, butadiynyl, pentadiynyl, hexadiynyl, heptadiynyl, octadiynyl, hexatriynyl, heptatriynyl, octatriynyl and isomer groups thereof and the like.

The "substituent" in the "aliphatic hydrocarbon group which may have a substituent(s)" represented by $R^a$, $R^{a'}$, $R^b$ and $R^{b'}$ includes a cyclic group (ring 1) which may have a substituent(s) and a substituent selected from the following third group, and 1 to 5 of these substituents may be substituted at substitutable positions.

<Third group>

(1) halogen, (2) $-OR^c$, (3) $-SR^c$, (4) $-NR^dR^{d'}$, (5) $-COOR^c$, (6) $-CONR^dR^{d'}$, (7) $-NR^cCOR^{c'}$, (8) $-NR^cSO_2R^{c'}$, (9) $-N(SO_2R^c)_2$, (10) $-SO_2R^e$, (11) $-SO_2NR^fR^{f'}R$ ,

wherein $R^c$, $R^{c'}$, $R^d$ and $R^{d'}$ have the same meanings as the above-described $R^a$, $R^a$, $R^b$ and $R^{b'}$, respectively; however, each of the $R^c$, $R^{c'}$ $R^d$ and $R^{d'}$ does not represent an aliphatic hydrocarbon group substituted with a substituent selected from this group (third group).

In the specification, the "cyclic group (ring 1) which may have a substituent(s)" represented by $R^a$, $R^{a'}$, $R^b$, $R^{b'}$ or $R^{N1}$, "cyclic group (ring 1) which may have a substituent(s)" as the "substituent" in the "aliphatic hydrocarbon group which may have a substituent(s)" represented by $R^a$, $R^{a'}$, $R^b$ or $R^{b'}$, and the "cyclic group (ring 1) which may have a substituent(s)" in the "C1-8 alkyl" which may be substituted with "cyclic group (ring 1) which may have a substituent(s)" represented by $R^{N1}$ have the same meanings, and the "cyclic group" in the "cyclic group (ring 1) which may have a substituent(s)" includes a carbon ring, a hetero ring and the like.

The carbon ring includes a C3-15 mano-, bi- or tri-cyclic carbon ring aryl which may be partially or entirely saturated. The "C3-15 mono-, bi- or tri-cyclic carbon ring aryl which may be partially or entirely saturated" includes cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene, anthracene and the like. In addition, the "C3-15 mono-, bi- or tri-cyclic carbon ring aryl which may be partially or entirely saturated" also includes a spiro-bonded bicyclic carbon ring and crosslinked bicyclic carbon ring, and their examples include spiro[4.4]nonane, spiro[4.5]decane, spiro[5.5]undecane, bicyclo[2.2.1]heptane, bicyclo[2.2.1]hept-2-ene, bicyclo[3.1.1]heptane, bicyclo[3.1.1]hept-2-ene, bicyclo[2.2.2]octane, bicyclo[2.2.2]oct-2-ene, adamantane, noradamantane and the like.

On the other hand, the hetero ring includes a 3- to 15-membered mono-, bi- or tri-cyclic hetero ring aryl which contains 1 to 4 nitrogen atoms, 1 to 3 oxygen atoms and/or 1 to 3 sulfur atoms, a hetero ring which is partially or entirely saturated, a spiro-bonded tri-cyclic hetero ring, a crosslinked tri-cyclic hetero ring and the like. The "3- to 15-membered mono-, bi- or tri-cyclic hetero ring aryl which contains 1 to 4 nitrogen atoms, 1 to 3 oxygen atoms and/or 1 to 3 sulfur atoms" includes pyrrole, imidazole, triazole, tetrazole, pyrazole, pyridine, pyrazine, pyrimidine, pyridazine, azepine, diazepine, furan, pyran, oxepine, thiophene, thiaine (thiopyran), thiepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, indole, isoindole, benzofuran, isobenzofuran, benzothiophene, isobenzothiophene, indazole, quinoline, isoquinoline, phthalazine, naphthyridine, quinoxaline, quinazoline, cinnoline, benzoxazole, benzothiazole, benzimidazole, benzoxepine, benzoxazepine, benzoxadiazepine, benzothiepine, benzothiazepine, benzothiadiazepine, benzoazepine, benzodiazepine, benzofurazan, benzothiadiazole, benzotriazole, carbazole, acridine, dibenzofuran, dibenzothiophene and the like. Also, those which are partially or entirely saturated among the "3- to 15-membered mono-, bi- or tri-cyclic (fused or spiro) hetero rings which contain 1 to 4 nitrogen atoms, 1 to 3 oxygen atoms and/or 1 to 3 sulfur atoms" include pyrroline, pyrrolidine, imidazoline, imidazolidine, pyrazoline, pyrazolidine, triazoline, triazolidine, tetrazoline, tetrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrothiophene, tetrahydrothiophene, dihydrothiaine (dihydrothiopyran), tetrahydrothiaine (tetrahydrothiopyran), dihydrooxazole, tetrahydrooxazole, dihydroisoxazole, tetrahydroisoxazole, dihydrothiazole, tetrahydrothiazole, dihydroisothiazole, tetrahydroisothiazole, dihydrooxadiazole, tetrahydrooxadiazole, dihydrothiodiazole, tetrahydrothiodiazole, tetrahydrooxadiazine, tetrahydrothiadiazine, tetrahydrooxazepine, tetrahydrooxadiazepine, pehydroxazepine, perhydrooxadiazepine, tetrahydrothiazepine, tetrahydrothiadiazepine, perhydrothiazepine, perhydrothiadiazepine, morpholine, thiomorpholine, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine,

tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, dihydrobenzoxazole, perhydrobenzoxazole, dihydrobenzothiazole, perhydrobenzothiazole, dihydrobenzimidazole, perhydrobenzimidazole, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolan, dioxane, dithiolan, dithian, benzodioxalan, benzodioxane, benzodithiolan, benzodithian, 2,4,6-trioxaspiro[bicyclo[3.3.0]octane-3,1'-cyclohexane], 1,3 -dioxolano[4,5-g]chromene, 2-oxabicyclo[2.2.1]heptane and the like.

As the "substituent" in the "cyclic group (ring 1) which may have a substituent(s)", a 5- to 10-membered monocyclic or bicyclic cyclic group is preferred, benzene, cyclohexane, cyclohexene, thiophene, pyrazole, isothiazole, thiazole, imidazole, furan, dihydropyrazole, quinoline, benzodioxane, dioxaindane, benzofuran, and pyridine are more preferred, and benzene, pyridine and pyrazole are particularly preferred.

The "substituent" in the "cyclic group (ring 1) which may have a substituent(s)" includes an aliphatic hydrocarbon group which may have a substituent(s), a cyclic group (ring 2) which may have a substituent(s), a substituent selected from the above-described first group, a substituent selected from the following fourth group and the like, and 1 to 5 of these substituents may be substituted at substitutable positions. In this case, the "aliphatic hydrocarbon group" as the "substituent" in the "aliphatic hydrocarbon group which may have a substituent(s)" has the same meaning as the above-described "linear or branched chain C1-8 hydrocarbon group". The "substituent" in the "aliphatic hydrocarbon group which may have a substituent(s)" includes substituents selected from a cyclic group (ring 2) which may have a substituent(s), a substituent selected from the above-described first group, a substituent selected from the following fourth group and the like, and 1 to 5 of these substituents may be substituted at substitutable positions.

<Fourth group>

(1) $-SR^e$, (2) $-SO_2R^e$, (3) $-SO_2NR^fR^{f'}$, (4) $-S(O)R^e$, (5) $-OR^e$, (6) $-OCOR^e$, (7) $-NR^eSO_2R^{e'}$, (8) $-NR^fR^{f'}$, (9) $-NR^eCOR^{e'}$, (10) $-NR^eCOOR^{e'}$, (11) $-NR^eCONR^fR^{f}$, (12) $-N(SO_2R^e)_2$, (13) $-COR^e$, (14) $-COOR^e$, (15) $-CONR^fR^{f'}$, (16) $-CONR^eCOR^{e'}$, (17) $-COCOOR^e$, (18) $-B(OR^e)_2$,

wherein $R^e$, $R^{e'}$, $R^f$ and $R^f$ each independently represents hydrogen, a cyclic group (ring 2) which may have a substituent(s) or an aliphatic hydrocarbon group which may have a substituent(s), or $R^f$ and $R^{f'}$ may be taken together with the adjacent nitrogen to represent (1) -C2-6 alkylene, (2) -(C2-6 alkylene)-O-(C2-6 alkylene)-, (3) -(C2-6 alkylene)-S-(C2-6 alkylene)-, (4) -(C2-6 alkylene)-$NR^{N2}$-(C2-6 alkylene)- (wherein $R^{N2}$ represents hydrogen, phenyl or C1-8 alkyl which may be substituted with phenyl).

In this case, the "aliphatic hydrocarbon group" in the "aliphatic hydrocarbon group which may have a substituent(s)" represented by $R^e$, $R^{e'}$, $R^f$ and $R^{f'}$ has the same meaning as the above-described "linear or branched chain C1-8 hydrocarbon group". The "substituent" in the "aliphatic hydrocarbon group which may have a substituent(s)" represented by $R^e$, $R^{e'}$, $R^f$ and $R^f$ includes substituents selected from a cyclic group (ring 2) which may have a substituent(s) and a substituent selected from the following fifth group, and 1 to 5 of these substituents may be substituted at substitutable positions.

<Fifth group>

(1) halogen, (2) $-OR^g$, (3) $-SR^g$, (4) $-NR^hR^{h'}$, (5) $-COOR^g$, (6) $-CONR^hR^{h'}$, (7) $-NR^gCOR^{g'}$, (8) $-NR^gSO_2R^{g'}$, (9) $-N(SO_2R^g)_2$,

wherein $R^g$, $R^{g'}$, $R^h$ and $R^{h'}$ have the same meanings as the above-described $R^e$, $R^{e'}$, $R^f$ and $R^{f'}$, respectively; however, each of the $R^g$, $R^{g'}$, $R^h$ and $R^{h'}$ does not represent an aliphatic hydrocarbon group substituted with a substituent selected from this group (fifth group).

In the specification, the "cyclic group (ring 2) which may have a substituent(s)" as the "substituent" in the "cyclic group (ring 1) which may have a substituent(s)", the "cyclic group (ring 2) which may have a substituent(s)" represented by $R^e$, $R^{e'}$, $R^f$ or $R^f$, and the "cyclic group (ring 2) which may have a substituent(s)" as the "substituent" in the "aliphatic hydrocarbon group which may have a substituent(s)" represented by $R^e$, $R^{e'}$ $R^f$ or $R^{f'}$ have the same meanings, and the "cyclic group" in the "cyclic group (ring 2) which may have a substituent(s)" has the same meaning as the above-described "cyclic group" in the "cyclic group (ring 1) which may have a substituent(s)" represented by $R^a$, $R^{a'}$, $R^b$, $R^{b'}$ or $R^{N1}$.

The "substituent" in the "cyclic group (ring 2) which may have a substituent(s)" includes substituents selected from an aliphatic hydrocarbon group which may have a substituent(s), a 3- to 8-membered monocyclic carbon ring or hetero ring which may have a substituent(s), a substituent selected from the above-described first group, a substituent selected from the following sixth group, and the like, and 1 to 5 of these substituents may be substituted at substitutable positions. In this case, the "aliphatic hydrocarbon group" as the "substituent " in the "aliphatic hydrocarbon group which may have a substituent(s)" has the same meaning as the above-described "linear or branched chain C1-8 hydrocarbon group". The "substituent" in the "aliphatic hydrocarbon group which may have a substituent(s)" includes substituents selected from a 3- to 8-membered monocyclic carbon ring or hetero ring which may have a substituent(s), a substituent selected from the above-described first group, a substituent selected from the following sixth group, and the like, and 1 to 5 of these substituents may be substituted at substitutable positions.

<Sixth group>

(1) $-SR^i$, (2) $-SO_2R^i$, (3) $-SO_2NR^jR^{j'}$, (4) $-S(O)R^i$, (5) $-OR^i$, (6) $-OCOR^i$, (7) $-NR^iSO_2R^{i'}$, (8) $-NR^jR^{j'}$, (9) $-NR^iCOR^{i'}$, (10) $-NR^iCOOR^{i'}$ (11) $-NR^iCONR^jR^{j'}$, (12) $-N(SO_2R^i)_2$, (13) $-COR^i$, (14) $-COOR^i$, (15) $-CONR^jR^{j'}$, (16) $-CONR^iCOR^{i'}$, (17) $-COCOOR^i$, (18) $-B(OR^i)_2$,

wherein $R^i$, $R^{i'}$, $R^j$ and $R^{j'}$ each independently represents hydrogen, a 3- to 8-membered monocyclic carbon ring or hetero ring which may have a substituent(s) or an aliphatic hydrocarbon group which may have a substituent(s), or $R^j$ and $R^{j'}$ may be taken together with the adjacent nitrogen to represent (1) -C2-6 alkylene (e.g., methylene, ethylene, trimethylene, tetramethylene, pentamethylene, hexamethylene and isomer groups thereof and the like)-, (2) -(C2-6 alkylene)-O-(C2-6 alkylene)-, (3) -(C2-6 alkylene)-S-(C2-6 alkylene)-, (4) -(C2-6 alkylene)-$NR^{N2}$-(C2-6 alkylene)-(wherein $R^{N2}$ represents the same meaning as described above).

In this case, the "aliphatic hydrocarbon group" in the "aliphatic hydrocarbon group which may have a substituent(s)" represented by $R^i$, $R^{i'}$, $R^i$ and $R^{j'}$ has the same meaning as the above-described "linear or branched chain C1-8 hydrocarbon group". The "substituent" in the "aliphatic hydrocarbon group which may have a substituent(s)" represented by $R^i$ $R^{i'}$, $R^j$ and $R^{j'}$ includes substituents selected from a 3- to 8-membered monocyclic carbon ring or hetero ring which may have a substituent(s) and a substituent selected from the following seventh group, and 1 to 5 of these substituents may be substituted at substitutable positions.

<Seventh group>

(1) halogen, (2) $-OR^k$, (3) $-SR^k$, (4) $-NR^mR^{m'}$, (5) $-COOR^k$, (6) $-CONR^mR^{m'}$, (7) $-NR^kCOR^{k'}$, (8) $-NR^kSO_2R^{k'}$, (9) $-N(SO_2R^k)_2$,

wherein $R^k$, $R^{k'}$, $R^m$ and $R^{m'}$ have the same meanings as the above-described $R^i$, $R^{i'}$, $R^j$ and $R^{j'}$, respectively; however, each of the $R^k$, $R^{k'}$, $R^m$ and $R^{m'}$ does not represent an aliphatic hydrocarbon group substituted with a substituent selected from this group (seventh group).

In the specification, the "C3-8 monocyclic carbon ring or hetero ring" in the "3- to 8-membered monocyclic carbon ring or hetero ring which may have a substituent(s)" includes a 3- to 8-membered monocyclic carbon ring aryl which may be partially or entirely saturated, a 3- to 8-membered monocyclic hetero ring aryl which contains 1 to 4 nitrogen atoms, 1 or 2 oxygen atoms and/or 1 or 2 sulfur atoms, a hetero ring which is partially or entirely saturated, and the like.

The "C3-8 monocyclic carbon ring aryl which may be partially or entirely saturated" includes cyclopropane, cyclobutane, cyclopentane, cyclohexane, cycloheptane, cyclooctane, cyclopentene, cyclohexene, cycloheptene, cyclooctene, cyclopentadiene, cyclohexadiene, cycloheptadiene, cyclooctadiene, benzene and the like. The "3- to 8-membered monocyclic hetero ring aryl which contains 1 to 4 nitrogen atoms, 1 or 2 oxygen atoms and/or 1 or 2 sulfur atoms" includes pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine and the like. The hetero ring which is partially or entirely saturated among the "3- to 8-membered monocyclic hetero ring aryl which contains 1 to 4 nitrogen atoms, 1 or 2 oxygen atoms and/or 1 or 2 sulfur atoms" includes aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazane, tetrahydrofurazane, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, pehydroxazepine, dihydrooxadiazepine, tetrahydrooxadizepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, dioxolan, dioxane, dithiolan, dithian and the like.

As the "3- to 8-membered monocyclic carbon ring or hetero ring" in the "3- to 8-membered monocyclic carbon ring or hetero ring which may have a substituent(s)", a 5 or 6-membered cyclic group is preferred, tetrahydropyran, tetrahydrothiopyran, piperidine, benzene, pyridine, pyrimidine, pyrazine, furan, oxazole, thiophene, pyrrole, thiazole and imidazole are more preferred, and benzene is particularly preferred.

In the present specification, the "substituent" in the "3- to 8-membered monocyclic carbon ring or hetero ring which may have a substituent(s)" includes substituents selected from C1-8 alkyl, a substituent selected from the above-described first group, a substituent selected from the following eighth group and the like.

<Eighth group>

(1) $-OR^n$, (2) $-NR^{\circ}R^{\circ'}$, (3) $-COOR^n$, (4) $-SR^n$, (5) $-CONR^{\circ}R^{\circ'}$,

wherein $R^n$, $R^{\circ}$ and $R^{\circ'}$ each independently represents hydrogen, phenyl or C1-8 alkyl which may be substituted with phenyl, or $R^{\circ}$ and $R^{\circ'}$ may be taken together with the adjacent nitrogen to represent (1) -C2-6 alkylene, (2) -(C2-6 alkylene)-O-(C2-6 alkylene)-, (3) -(C2-6 alkylene)-S-(C2-6 alkylene)-, (4) -(C2-6 alkylene)-$NR^{N2}$-(C2-6 alkylene)- (wherein $R^{N2}$ represents the same meaning as described above).

In the specification, the "cyclic group which may have a substituent(s)" as the "substituent" in the "aliphatic hydrocarbon group which may have a substituent(s)" represented by $R^1$ has the same meaning as the "cyclic group (ring 1) which may have a substituent(s)" represented by $R^a$, $R^{a'}$, $R^b$, $R^{b'}$ or $R^{N1}$. As the "cyclic group" in the "cyclic group which may have a substituent(s)", a 5- to 10-membered monocyclic or bicyclic cyclic group is preferred, benzene, cyclohexane, cyclohexene, thiophene, pyrazole, isothiazole, thiazole, imidazole, furan, dihydropyrazole, quinoline, benzodioxane, dioxaindane, benzofuran, and pyridine are more preferred, and benzene, pyridine and pyrazole are particularly preferred.

In the present invention, $R^1$ is preferably an aliphatic hydrocarbon group which may have a substituent(s), more preferably C1-6 alkyl which may have a 3- to 15-membered mono- or bi-cyclic group which may have a substituent(s), as its substituent(s). The C1-6 alkyl which may have a 3- to 15-membered mono- or bi-cyclic group which may have a substituent(s), as its substituent(s), is preferably -(C1-6 alkyl)-(benzene which may have a substituent(s)), -(C1-6 alkyl)-(pyridine which may have a substituent(s)), -(C1-6 alkyl)-(pyrazole which may have a substituent(s)), -(C1-6 alkyl)-(C4-6 cycloalkyl which may have a substituent(s)), -(C1-6 alkyl)-(C4-6 cycloalkenyl which may have a substituent(s)), -(C1-6 alkyl)-(thiazole which may have a substituent(s)), -(C1-6 alkyl)-(furan which may have a substituent(s)), -(C1-6 alkyl)-(isoxazole which may have a substituent(s)), -(C1-6 alkyl)-(thiophene which may have a substituent(s)), -(C1-6 alkyl)-(quinoline which may have a substituent(s)), -(C1-6 alkyl)-(benzodioxane which may have a substituent(s)), -(C1-6 alkyl)-(dioxaindane which may have a substituent(s)), -(C1-6 alkyl)-(benzofuran which may have a substituent(s)), -(C1-6 alkyl)-(oxadiazole which may have a substituent(s)), -(C1-6 alkyl)-(pyrrole which may have a substituent(s)), -(C1-6 alkyl)-(dihydrobenzodioxane which may have a substituent(s)), -(C1-6 alkyl)-(pyrazole which may have a substituent(s)), -(C1-6 alkyl)-(imidazole which may have a substituent(s)), -(C1-6 alkyl)-(isothiazole which may have a substituent(s)), -(C1-6 alkyl)-(dihydropyrazole which may have a substituent(s)), and more preferably benzyl or one represented by the following formula:

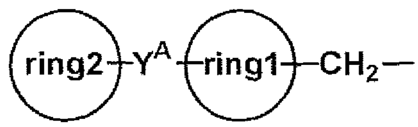

wherein ring 1 represents the "cyclic group (ring 1) which may have a substituent(s)", ring 2 represents the "cyclic group (ring 2) which may have a substituent(s)", $Y^A$ represents a bond, $-CH_2-$, $-CH_2CH_2-$, -O-, -S-, -CO-, -S(O)-, $-SO_2-$, -CH(OH)-, $-NR^f-$, $-CONR^f$, $-NR^eCO-$, $-CH_2O-$, $-OCH_2-$, -CH=CH-, $-CONR^fCH_2-$, $-CH_2CONR^h$, $-CH_2NR^gCO-$, $-NR^eCOCH_2-$, $-NR^eSO_2-$, $-SO_2NR^f-$, $-SO_2NR^fCH_2-$, $-CH_2SO_2NR^h$, $-CH_2NR^gSO_2-$ or $-NR^eSO_2CH_2-$, and all of $R^f$, $R^e$, $R^h$ and $R^g$ have the same meanings as those defined above.

In this case, $Y^A$ is preferably a bond, -O-, $-CH_2-$, -CO- or the like. As ring 1 and ring 2, "5- to 10-membered carbon ring aryl or hetero ring aryl" and the like are preferred. The "5- to 10-membered carbon ring aryl or hetero ring aryl" is preferably "5- or 6-membered carbon ring aryl or hetero ring aryl" or the like, and preferred examples include benzene, pyrrole, imidazole, triazole, tetrazole, pyrazole, pyridine, pyrazine, pyrimidine, pyridazine, triazine, furan, thiophene, oxazole, isoxazole, thiazole, isothiazole, furazane, oxadiazole and thiadiazole rings. As described above, these cyclic groups may have a substituent(s), and those in which acidic group such as carboxyl which may be protected, amide or sulfonamide is substituted on the ring 2 are particularly preferred. In addition, the substituents of ring 1 and ring 2 include an aliphatic hydrocarbon group which may have a substituent(s), alkoxy, carboxyl, alkanoylamide and the like, and more preferred substituents are an aliphatic hydrocarbon group, alkoxy and the like. In the present invention, $R^1$ having a combination thereof is more desirable. Particularly preferred are 1,3-thiazol-2-ylmethyl, 1,5-dimethyl-3-oxo-2-phenyl-2,3-dihydro-1H-pyrazol-4-ylmethyl, 2-(4-isopropyl-benzyl)-propyl, 2,4,6-trimethoxybenzyl, 2,4-dimethoxybenzyl, 2,6-dimethoxybenzyl, 2-phenylimidazol-5-ylmethyl, 2-phenylethyl, 2-benzyloxybenzyl, 2-methoxybenzyl, 3-(furan-2-yl)-2-propenyl, 3,5-dimethyl-1-phenyl-1H-pyrazol-4-ylmethyl, 3-cyanobenzyl, 3-phenylpyrazol-4-ylmethyl, 3-phenylpropyl, 3-phenoxybenzyl, 4-(3-(N,N-dimethylamino)propyloxy)benzyl, 1, 4-(4-hydroxy-4-methylpentyl)cyclohex-3-ene-1-ylmethyl, 4-(4-methylsulfonylamino)-phenoxybenzyl, 4-(N,N-dimethylamino)benzyl, 4-(dihydroxyboryl)benzyl, 4-(methylcarbonylamino)benzyl, 4-chlorobenzyl, 4-phenylbenzyl, 4-phenoxybenzyl, 4-fluorobenzyl, 6-methyl-2,2-dimethylcyclohex-1-en-1-ylethyl, quinoline-2-ylmethyl, dioxaindan-4-ylmethyl, cyclopropane-1-ylmethyl, thiophen-2-ylmethyl, furan-2-ylmethyl, benzyl, and benzodioxan-6-ylmethyl, benzafuran-2-ylmethyl.

In the specification, the "3- to 15-membered nitrogen-containing mono-, bi- or tri-cyclic hetero ring" in the "3- to 15-membered nitrogen-containing mono-, bi- or tri-cyclic hetero ring which may have a substituent(s)" represented by ring A includes mono-, bi- or tri-cyclic hetero rings which contain at least one nitrogen atom other than a carbon atom and may further contain 1 to 3 hetero atoms selected from nirtrogen, oxygen and sulfur. The "3- to 15-membered nitrogen-containing heterocyclic ring" includes a "3- to 15-membered nitrogen-containing unsaturated hetero ring" and a "3- to 15-membered nitrogen-containing saturated hetero ring".

The "3- to 15-membered nitrogen-containing unsaturated hetero ring" includes pyrroline, imidazoline, triazoline, tetrazoline, pyrazoline, dihydropyridine, tetrahydropyridine, dihydropyrazine, tetrahydropyrazine, dihydropyrimidine, tetrahydropyrimidine, dihydropyridazine, tetrahydropyridazine, dihydroazepine, tetrahydroazepine, dihydrodiazepine, tetrahydrodiazepine, dihydrooxazole, dihydroisoxazole, dihydrothiazole, dihydroisothiazole, dihydrofurazan, dihydrooxadiazole, dihydrooxazine, dihydrooxadiazine, dihydrooxazepine, tetrahydrooxazepine, dihydrooxadiazepine, tetrahydrooxadiazepine, dihydrothiadiazole, dihydrothiazine, dihydrothiadiazine, dihydrothiazepine, dihydrothiadiazepine, tetrahydrothiadiazepine, indoline, isoindoline, dihydroindazole, dihydroquinoline, tetrahydroquinoline, dihydroisoquinoline, tetrahydroisoquinoline, dihydrophthalazine, tetrahydrophthalazine, dihydronaphthyridine, tetrahydronaphthyridine, dihydroquinoxaline, tetrahydroquinoxaline, dihydroquinazoline, tetrahydroquinazoline, dihydrocinnoline, tetrahydrocinnoline, dihydrobenzoxazine, dihydrobenzothiazine, pyrazinomorpholine, dihydrobenzoxazole, dihydrobenzothiazole, dihydrobenzimidazole, dihydrobenzazepine, tetrahydrobenzazepine, dihydrobenzodiazepine, tetrahydrobenzodiazepine, dihydrobenzoxazepine, tetrahydrobenzoxazepine, dihydrocarbazole, tetrahydrocarbazole, dihydroacridine, tetrahydroacridine and the like.

The "3- to 15-membered nitrogen-containing saturated hetero ring" includes aziridine, azetidine, azocane, pyrrolidine, imidazolidine, triazolidine, tetrazolidine, pyrazolidine, piperidine, piperazine, perhydropyrimidine, perhydropyridazine, perhydroazepine, perhydrodiazepine, tetrahydrooxazole (oxazolidine), tetrahydroisoxazole (isoxazolidine), tetrahydrothiazole (thiazolidine), tetrahydroisothiazole (isathiazolidine), tetrahydrofurazan, tetrahydrooxadiazole (oxadiazolidine), tetrahydrooxazine, tetrahydrooxadiazine, perhydrooxazepine, perhydrooxadiazepine, tetrahydrothiadiazole (thiadiazolidine), tetrahydrothiazine, tetrahydrothiadiazine, tetrahydrothiazepine, perhydrothiazepine, perhydrothiadiazepine, morpholine, thiomorpholine, perhydroindazole, perhydroquinoline, perhydroisoquinoline, perhydrophthalazine, perhydronaphthyridine, perhydroquinoxaline, perhydroquinazoline, perhydrocinnoline, perhydrobenzoxazole, perhydrobenzothiazole, perhydrobenzimidazole, perhydrocarbazole, perhydroacridine,

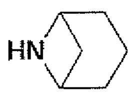 , 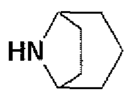 , 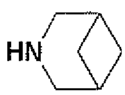 , 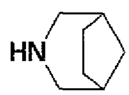 , or 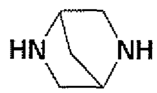

and the like.

The "3- to 15-membered nitrogen-containing mono-, bi- or tri-cyclic hetero ring" represented A is preferably a "4- to 8-membered nitrogen-containing mono-cyclic hetero ring" or a "9- to 15-membered nitrogen-containing bi- or tri-cyclic hetero ring".

Examples include:

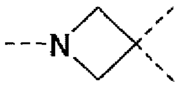 , 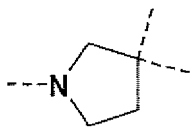 , 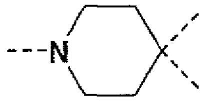 , 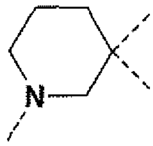 ,

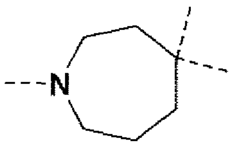 , 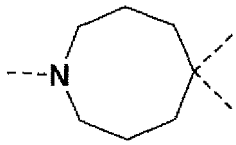 , 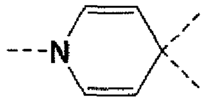 , 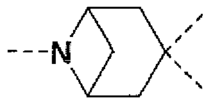 ,

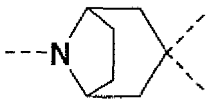 , 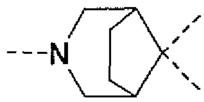 , 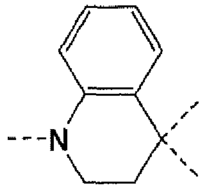 , 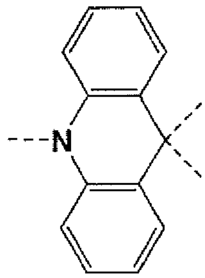

and the like.

However, when ring A is

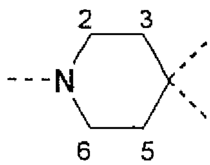 ,

ring A has a substituent(s) other than $R^1$. That is, when ring A is piperidine, ring A has a substituent(s) on at least one position of 2-, 3-, 5- and 6- positions. In these compounds, the dotted line binding to the nitrogen atom represents $R^1$-binding position, and other dotted lines represent a spiro bond to ring B.

Ring A may have 1 to 5 substituents at substitutable positions. The "substituent" in ring A includes a substituent selected from the following ninth group, a linear or branched chain C1-8 hydrocarbon group which may have 1 to 5 substituents selected from the following ninth group, and the like.

<Ninth group>

(1) halogen (e.g., chlorine, bromine, fluorine, iodine), (2) $-OR^x$, (3) $-COR^x$, (4) $-CONR^xR^{x'}$, (5) $-COOR^x$, (6) $-NR^xR^{x'}$, (7) $-NR^xCOR^{x'}$, (8) $-SO_2R^y$, (9) $-SO_2NR^xR^{x'}$, (10) $-NR^xSO_2R^y$, (11) oxo, (12) $=NR^1$

wherein $R^x$ and $R^{x'}$ each independently represent hydrogen or a linear or branched chain C1-8 hydrocarbon group, $R^y$ represents linear or branched chain C1-8 hydrocarbon group, and $R^t$ represents hydrogen atom, a linear or branched chain C1-8 hydrocarbon group or a hydroxyl group which may be protected.

Herein, the "linear or branched chain C1-8 hydrocarbon group" has the same meaning as described above.

The hydroxyl group which may be protected has the same meaning as $-OR^a$ (wherein the symbol has the same meaning as described above) in the above second group.

In the present invention, the substituent of ring A is preferably methyl, methoxy or carboxyl.

In the specification, the "substituent" in the ring B which may have a substituent(s) includes an aliphatic hydrocarbon group which may have a substituent(s), a cyclic group which may have a substituent(s), a hydroxyl group which may be protected, a carboxyl group which may be protected, a carbamoyl group which may be substituted, or

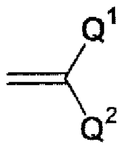

wherein $Q^1$ and $Q^2$ each independently represents hydrogen, an aliphatic hydrocarbon group which may have a substituent(s), a cyclic group which may have a substituent(s), a hydroxyl group which may be protected, a carboxyl group which may be protected, or a carbamoyl group which may be substituted.

Herein, the hydroxyl group which may be protected, the carboxyl group which may be protected and the carbamoyl group which may be substituted have the same meanings as $-OR^a$, $-COOR^a$, $-CONR^bR^{b'}$ (wherein the symbols have the same meanings as those described above) described in the above second group. Also, the "aliphatic hydrocarbon group" in the "aliphatic hydrocarbon group which may have a substituent(s)" includes a "linear or branched chain C1-8 hydrocarbon group". The "linear or branched chain C1-8 hydrocarbon group" has the same meaning as described above. The "substituent" in this "aliphatic hydrocarbon group which may have a substituent(s)" has the same meaning as the "substituent" in the "aliphatic hydrocarbon group which may have a substituent(s)" represented by $R^1$. Herein, the "linear or branched chain C1-8 hydrocarbon group" has the same meaning as described above. The "cyclic group which may have a substituent(s)" has the same meaning as the "cyclic group (ring 1) which may have a substituent(s)" in the above second group.

In the specification, $R^2$ and $R^3$ each independently represents hydrogen, an aliphatic hydrocarbon group which may have a substituent(s), a cyclic group which may have a substituent(s), a hydroxyl group which may be protected, a carboxyl group which may be protected, a carbamoyl group which may be substituted, or the like.

Herein, the hydroxyl group which may be protected, the carboxyl group which may be protected and the carbamoyl group which may be substituted have the same meanings as $-OR^a$, $-COOR^a$, $-CONR^bR^{b'}$ (wherein the symbols have the same meanings as those described above) described in the above second group.

Also, the "aliphatic hydrocarbon group" in the "aliphatic hydrocarbon group which may have a substituent(s)" includes a "linear or branched chain C1-8 hydrocarbon group". The "linear or branched chain C1-8 hydrocarbon group" has the same meaning as described above. The "substituent" in this "aliphatic hydrocarbon group which may have a substituent(s)" has the same meaning as the "substituent" in the "aliphatic hydrocarbon group which may have a substituent(s)" represented by $R^1$.

Herein, the "linear or branched chain C1-8 hydrocarbon group" has the same meaning as described above. The "cyclic group which may have a substituent(s)" has the same meaning as the "cyclic group (ring 1) which may have a substituent(s)" in the above second group.

In the present invention, the substituent on ring B is preferably a 4- to 6-membered carbon ring which may have a substituent(s) or a C1-6 aliphatic hydrocarbon group which may have a substituent(s), and more preferably -(C1-6 alkyl which may have a substituent(s)), -(C2-6 alkenyl which may have a substituent(s)), -(C2-6 alkynyl which may have a substituent(s)), -(benzene which may have a substituent(s)), -(C1-6 alkyl which may have a substituent(s))-(C4-6 cycloalkyl which may have a substituent(s)), -(C1-6 alkyl which may have a substituent(s))-(C4-6 cycloalkenyl which may have a substituent(s)), -(C1-6 alkyl which may have a substituent(s))-(4- to 6-membered hetero cycle which may have a substituent(s)), -(C1-6 alkyl which may have a substituent(s))-(benzene which may have a substituent(s)), or -(C1-6 alkyl which may have a substituent(s))-NHCOO-(C1-6 alkyl which may have a substituent(s))-(benzene which may have a substituent(s)), and more preferably -(C1-6 alkyl which may have a substituent(s)), -(C1-4 alkyl which may have a substituent(s))-(C4-6 cycloalkyl), -(C1-6 alkyl which may have a substituent(s))-(C4-6 cycloalkenyl which may have a substituent(s)), -(C1-6 alkyl which may have a substituent(s))-(tetrahydropyranyl), or -(C1-4 alkyl which may have a substituent(s))-(benzene). Particularly, propyl, butyl, sec-butyl, cyclobutylmethyl, cyclopentylmethyl, cyclohexylmethyl, cyclobutyl(hydroxy)methyl, cyclopentyl(hydroxy)methyl, cyclohexyl(hydroxy) methyl, tetrahydropyran-4-yl(hydroxy) methyl, benzyl and phenylethyl are preferred.

In the present invention, the "carboxyl group which may be protected" which is exemplified as the preferred substituent of the cyclic ring (ring 2) which may have a substituent(s) includes $-COOR^a$ (wherein the symbols have the same meanings as those described above) in the above second group.

Unless otherwise specified, all isomers are included in the present invention. For example, alkyl, alkenyl, alkynyl, alkoxy alkylthio, alkylene, alkenylene and alkynylene groups include straight or branched ones. In addition, isomers on double bond, ring, fused ring (E-, Z-, cis-, trans-isomer), isomers generated from asymmetric carbon atoms (R-, S-isomer, α-, β-configuration, enantiomer, diastereomer), optically active isomer (D-, L-, d-, 1-isomer), polar compounds generated by chromatographic separation (more polar compound, less polar compound), equilibrium compounds, rotational isomer, mixtures thereof at voluntary ratios and racemic mixtures are also included in the present invention.

In the present invention, unless otherwise specified, as will be apparent to those skilled in the art, a symbol 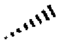 represents bonding to back of the paper (that is, α-configuration), 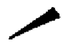 represents bonding to front of the paper (that is, β-configuration), 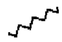 represents α-configuration, β-configuration or mixture thereof and 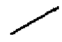 represents mixture of α-configuration and β-configuration.

The compounds represented by formula (I) may be converted into salts by conventional means. As the salts, pharmaceutically acceptable salts are preferred.

The pharmaceutically acceptable salts include alkali metal salts, alkali earth metal salts, ammonium salts, amine salts, acid addition salts, and the like.

Water-soluble salts are preferred as the pharmaceutically acceptable salts. Suitable salts, for example, include salts of alkali metals (e.g. potassium and sodium), salts of alkaline earth metals (e.g. calcium and magnesium), ammonium salts (quaternary ammonium salts, etc.), and salts of pharmaceutically acceptable organic amines (e.g. tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine and N-methyl-D-glucamine).

Preferred acid addition salts are water-soluble salts. Suitable acid addition salts, for example, include: salts of inorganic acids e.g. hydrochloride, hydrobromide, hydroiodide, sulfate, phosphate, nitrate, and salts of organic acids e.g. acetate, lactate, tartrate, benzoate, citrate, methanesulfonate, benzenesulfonate, toluenesulfonate, isethionate, glucuronate and gluconate.

The compounds represented by formula (1) and salts thereof may be converted into the corresponding solvates.

Preferred solvates are non-toxic and water-soluble salts. Suitable solvates, for example, include: solvates of water and alcohol (e.g. ethanol) and the like

All of the compounds represented by formula (I) or pharmaceutically acceptable salts thereof are preferable, concretely, the compounds described in the example or pharmaceutically acceptable salts thereof

Also, the salts include quaternary ammonium salts. The quaternary ammonium salts are the compounds wherein nitrogen of the compounds represented by formula (I) is quarternalized by $R^0$.

$R^0$ is C1-8 alkyl or C1-8 alkyl substituted with phenyl.

The compounds of the present invention can be converted into N-oxides by arbitrary method. The N-oxides are compounds wherein nitrogen of the compounds represented by formula (I) is oxidized.

Also, a prodrug of the compound of formula (I) (compound (I)) means a compound which is converted to the compound (I) by reaction with an enzyme, gastric acid or the like in the living body. For example, with regard to a prodrug of the compound (I), when the compound (I) has an amino group, compounds in which the amino group is, for example, acylated, alkylated or phosphorylated (e.g., compounds in which the amino group of the compound (I) is eicosanoylated, alanylated, pentylaminocarbonylated, (5-methyl-2-oxo-1,3-dioxolen-4-yl)methoxycarbonylated, tetrahydrofuranylated, pyrrolidyimethylated, pivaloyloxymethylated, acetoxymethylated, tert-butylated, etc.); when the compound (I) has a hydroxyl group, compounds where the hydroxyl group is, for example, acylated, alkylated, phosphorylated or borated (e.g., compounds in which the hydroxyl group of the compound (I) is acetylated, palmitoylated, propanoylated, pivaloylated, succinylated, fumarylated, alanylated or dimethylaminomethylcarbonylated); and that the carboxyl group of the compound (I) is, for example, esterified or amidated (e.g., compounds in which the carboxyl group of the compound (I) is made into ethyl ester, phenyl ester, phenylethyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester or methylamide). Those compounds may be produced by a known method *per se.* The prodrug of the compound (I) may be either a hydrate or a non-hydrate.

Processes for the preparation of the compound of the present invention:

The compound represented by formula (I) can be prepared by methods which properly improved and combined known methods, such as methods described below, methods described in Examples or methods described in *Comprehensive Organic Transformations:A Guide to Functional Group Preparations,* 2nd Edition (Richard C. Larock, John Wiley & Sons Inc, 1999). In each method described below, a starting material can be used as a salt thereof. An example of the salt includes a salt of formula (I) described above.

Among the compounds of the present invention represented by formula (I), compounds in which each of $R^2$

and $R^3$ is hydrogen, i.e., compounds of formula (I-1)

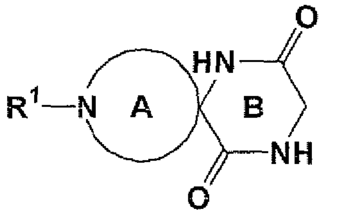

(I-1)

(wherein all symbols have the same meanings as the above-described 1) may be prepared by cyclization of a compound of formula (II)

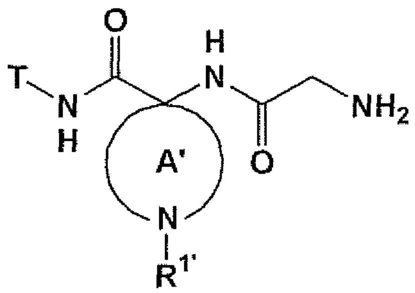

(II)

(wherein T is C1-4 alkyl, C5-6 mono-cyclic carbon ring or C1-4 alkyl substituted with C5-6 mono-cyclic carbon ring or 5- or 6-membered mono-cyclic hetero ring containing 1-2 nitrogen atoms and/or one oxygen atom, $R^{1'}$ and ring A' have the same meanings as $R^1$ and ring A, respectively, provided that when $R^{1'}$ or ring A contains carboxyl, hydroxyl, amino or thiol, such a group should be protected if such protection is necessary, and other symbols have the same meanings as those described above), followed by removal of a protecting group, if necessary.

The cyclization is known *per se* and can be carried out by, for example, heating in an organic solvent (dichloroethane, toluene, ethyl acetate etc.), with tertiary amine (triethylamine or diisopropylethylamine *etc.)* or acid (acetic acid or trifluoroacetic acid *etc.*)*,* or without tertiary amine or acid at 60-120°C. This cyclization reaction is carried out with the cleavage of T group.

Also, the compound may be converted to a desired salt thereof by a known method after the reaction, if necessary.

The removal of a protective group is known and carried out by the following method.

A protecting group of carboxyl includes, for example, methyl, ethyl, allyl, t-butyl, trichloroethyl, benzyl (Bn) or phenacyl *etc.*

A protecting group of hydroxy includes, for example, methyl, trityl, methoxymethyl (MOM), 1-ethoxyethyl (EE), methoxyethoxymethyl (MEM), 2-tetrahydropyranyl (THP), trimethylsilyl (TMS), triethylsilyl (TES), t-butyldimethylsilyl (TBDMS), t-butyldiphenylsilyl (TBDPS), acetyl (Ac) pivaloyl, benzoyl, benzyl (Bn), p-methoxybenzyl, allyloxycarbonyl (Alloc) or 2,2,2-trichloroethoxycarbonyl (Troc) *etc.*

A protecting group of amino includes, for example, benzyloxycarbonyl, t-butoxycarbonyl, allyloxycarbonyl (Alloc), 1-methyl-1-(4-biphenyl)ethoxycarbonyl (Bpoc), trifluoroacetyl, 9-fluorenylmethoxycarbonyl, benzyl (Bn), p-methoxybenzyl, benzyloxymethyl (BOM) or 2-(trimethylsilyl)ethoxymethyl (SEM) *etc.*

A protecting group of thiol includes, for example, benzyl, methoxybenzyl, methoxymethyl (MOM), 2-tetrahydropyranyl (THP), diphenylmethyl or acetyl (Ac).

The protecting group of carboxyl, hydroxy, amino or thiol includes the above one, and in addition to the other protecting group which is removable selectively and easily, for example, one described in T. W. *Greene et al.*, *Protective Groups in Organic Synthesis,* Third Edition, Wiley-Interscience, New York, 1999.

The removal of a protecting group of carboxyl, hydroxy, amino or thiol is known *per se.* For example, it is

(1) the alkaline hydrolysis,
(2) the removal of a protecting group in an acidic condition,
(3) the removal of a protecting group by hydrogenolysis,

(4) the removal of a protecting group containing silyl,
(5) the removal of a protecting group using metal or
(6) the removal of a protecting group using metal complex *etc.*

The concrete descriptions of these methods are as follows:

(1) The removal of a protecting group by alkaline hydrolysis condition may be carried out, for example, in an organic solvent (methanol, tetrahydrofuran or dioxane *etc.)* with hydroxide of alkaline metal (sodium hydroxide, potassium hydroxide or lithium hydroxide *etc.*), hydroxide of alkaline earth metal (barium hydroxide or calcium hydroxide *etc.*) or carbonate (sodium carbonate or potassium carbonate *etc.*), or an aqueous solution thereof or a mixture thereof at 0-100°C.
(2) The removal of a protecting group in an acidic condition may be carried out, for example, in an organic solvent (dichloromethane, chloroform, dioxane, ethyl acetate or anisole etc.), organic acid (acetic acid, trifluoroacetic acid, methanesulfonic acid or p-toluenesulfonic acid *etc.)* or inorganic acid (hydrochloric acid or sulfuric acid *etc.),* or a mixture thereof (hydrogen bromide/acetic acid *etc.)* at 0-40°C.
(3) The removal of a protecting group by hydrogenolysis may be carried out, for example, in a solvent (ether (tetrahydrofuran, dioxane, dimethoxyethane or diethylether etc.), alcohol (methanol or ethanol etc.), benzene (benzene or toluene etc.), ketone (acetone or methylethylketone etc.), nitrile (acetonitrile *etc.),* amide (dimethylformamide etc.), water, ethyl acetate, acetic acid, or a mixture thereof *etc.)* in the presence of a catalyst (palladium on carbon, palladium black, palladium hydroxide, platinum oxide, or Raney nickel *etc.),* at an atmospheric or positive pressure under atmosphere of hydrogen or in the presence of ammonium formate at 0-200°C.
(4) The removal of a protecting group containing silyl may be carried out, for example, in an organic solvent which can be mixed with water (tetrahydrofuran or acetonitrile etc.), with tetrabutylammoniumfluoride at 0-40°C.
(5) The removal of a protecting group using metal may be carried out, for example, in an acidic solvent (acetic acid or buffer solution of pH 4.2-7.2, or mixture of the above solution and an organic solvent such as tetrahydrofuran *etc.*)*,* in the presence of zinc powder, if necessary, with sonication at 0-40°C.
(6) The removal of a protecting group using metal complex may be carried out, for example, in an organic solvent (dichloromethane, dimethylformamide, tetrahydrofuran, ethyl acetate, acetonitrile, dioxane or ethanol *etc.)* or water, or mixture thereof in the presence of a trap reagent (tributyltin hydride, triethylsilane, dimedone, morpholine, diethylamine or pyrrolidine etc.), and an organic acid (acetic acid, formic acid, 2-ethylhexanoic acid *etc.*) and/or an organic acid salt (sodium 2-ethylhexanoate or potassium 2-ethylhexanoate *etc.*) in the presence or absence of phosphine reagent (triphenylphosphine *etc.)* with metal complex (tetrakis(triphenylphosphine)palladium(0), bis(triphenylphosphine)palladium(II) dichloride, palladium(II) acetate, tris(triphenylphosphine)chlororhodium(I) *etc.*) at 0-40°C.

Furthermore, as methods other than the above methods, the removal of a protecting group may be prepared by, for example, the methods described in T. W. Greene, *Protective Groups in Organic Synthesis,* Wiley, New York, 1999.
As well known to the person in the art, the aimed compounds of the present invention may be prepared easily by choice of these methods.
Furthermore, among the compounds represented by formula (I), compounds wherein $R^1$ is an aliphatic hydrocarbon group which may be substituted, each of $R^2$ and $R^3$ is hydrogen, and $R^1$ binds to nitrogen via $-CH_2-$, i.e., compounds represented by formula (I-A)

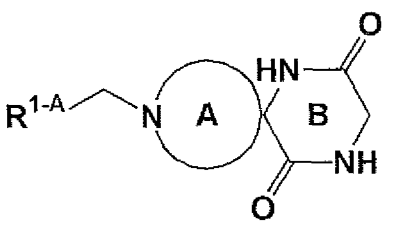
**(I-A)**

(wherein $R^{1-A}$ is an aliphatic hydrocarbon group which may have a substituent(s), provided that $R^{1-A}$ represents an aliphatic hydrocarbon group in which one carbon atom is decreased from the main chain of $R^1$ (the carbon number in the main chain of $R^{1-A}$ = the carbon number in the main chain of $R^1$ - 1), and other symbols have the same meanings as described above) may be prepared by reductive amination of a compound represented by formula (III)

$R^{1'-A}$—CHO **(III)**

(wherein $R^{I1\text{-}A}$ has the same meaning as $R^{1\text{-}A}$, provided that when $R^{1,\text{-}A}$ contains carboxyl, hydroxyl, amino or thiol, such a group should be protected if such protection is necessary, and other symbols have the same meanings as those described above) with a compound represented by formula (IV)

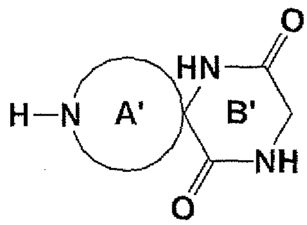

(IV)

(wherein ring B' has the same meaning as ring B, provided that when ring B' contains carboxyl, hydroxyl, amino or thiol, such a group should be protected if such protection is necessary, and other symbols have the same meanings as those described above), followed by removal of a protecting group, if necessary.

The reductive amination is known *per se* and can be carried out, for example, in an organic solvent (dichloroethane, dichloromethane, dimethylformamide or acetic acid, or a mixture thereof *etc.*) in the presence of a reducing agent (sodium triacetoxyborohydride, sodium cyanoborohydride or sodium borohydride *etc.*) at 0-40°C.

Moreover, the compounds represented by formula (I) may be prepared by reacting a compound represented by formula (V)

**$R^{1'}$—X (V)**

(wherein X is a leaving group (e.g., halogen, mesilate, tosylate, *etc.*), and other symbol have the same meanings as those described above) with a compound represented by formula (IV), followed by removal of a protecting group, if necessary.

The reaction is known *per se* and can be carried out, for example, in an organic solvent (e.g., dimethylsulfoxide, dimethylformamide, tetrahydrofuran, *etc.*) in the presence or absence of alkali (potassium carbonate, sodium carbonate, *etc.*) and sodium iodide or potassium iodide at 100-150°C.

Among the compounds represented by formula (I), compounds wherein $R^1$ represents C3-15 mono-, bi- or tri-cyclic carbon ring aryl which is partially or entirely saturated or 3- to 15-membered mono-, bi- or tri-cyclic hetero ring aryl which is partially or entirely saturated and has a hetero atom(s) selected from 1 to 4 nitrogen atoms, 1 to 2 oxygen atoms and/or 1 to 2 sulfur atoms, and each of $R^2$ and $R^3$ is hydrogen, i.e., compounds represented by formula (I-B)

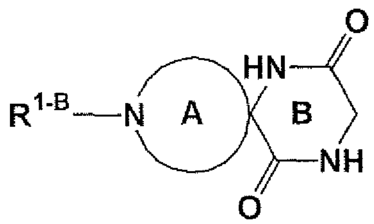

(I-B)

(wherein $R^{1\text{-}B}$ is ring 1 and is C3-15 mono-, bi- or tri-cyclic carbon ring aryl which is partially or entirely saturated or 3- to 15-membered mono-, bi- or tri-cyclic hetero ring aryl which is partially or entirely saturated and has a hetero atom(s) selected from 1 to 4 nitrogen atoms, 1 to 2 oxygen atoms and/or 1 to 2 sulfur atoms, and other symbols have the same meanings as those described above) may be prepared by reductive amination of a compound represented by formula (VI)

**$R^{1\text{-}B}$=O (VI)**

(wherein all symbols have the same meanings as those described above) with a compound represented by formula (IV).

The reductive amination is known *per* se and can be carried out, for example, in an organic solvent (dichloroethane, dichloromethane, dimethylformamide or acetic acid, or a mixture thereof *etc.*) in the presence of a reducing agent (sodium triacetoxyborohydride, sodium cyanoborohydride or sodium borohydride *etc.*) at 0-40°C.

Among the compounds of the present invention represented by formula (I), compounds wherein at least one

nitrogen is a quaternary ammonium salt, i.e., compounds represented by formula (I-2)

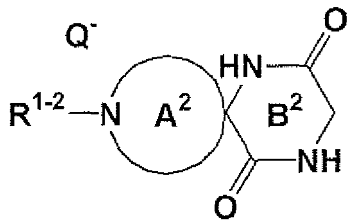

**(I-2)**

(wherein $R^{1-2}$, ring $A^2$ and ring $B^2$ have the same meanings as $R^1$, ring A and ring B, respectively, provided that at least one nitrogen atom is a quaternary ammonium salt, and Q is halogen) may be prepared by reacting a compound represented by formula (I) with a compound represented by formula (VII)

**$R^0$—Q** **(VII)**

(wherein R° is C1-4 alkyl, or C1-4 alkyl substituted with phenyl and Q is halogen.).

The reaction is known *per se* and can be carried out, for example, in an organic solvent (acetone, dimethylformamide or methyl ethyl ketone *etc.)* at 0-40°C.

Among the compounds of the present invention represented by formula (I), the compounds where at least one nitrogen represents N-oxide, i.e., the compounds of formula (1-3)

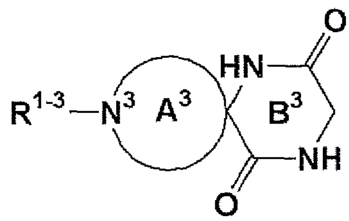

**(I-3)**

(wherein $R^{1-3}$, ring $A^3$ and ring $B^3$ have the same meanings as $R^1$, ring A and ring B, respectively, and $N^3$ represents nitrogen, provided that at least one nitrogen is N-oxide) may be prepared by oxidation of a compound represented by formula (I).

The oxidation is known *per se* and can be carried out, for example, in a suitable organic solvent (dichloromethane, chloroform, benzene, hexane or t-butylalcohol *etc.)* in the presence of an excessive oxidizing reagent (hydrogen peroxide, sodium periodate, acyl nitrite, sodium perborate, peroxidized acid (for example, 3-chloroperbenzoic acid or peracetic acid *etc.),* OXONE (brand name, potassium peroxymonosulfate.), potassium permanganate or chromic acid *etc.*) at 20-60°C.

The compounds of formula (II) and formula (IV) may be prepared by the method according to the following Reaction Scheme 1.

Reaction Scheme (1)

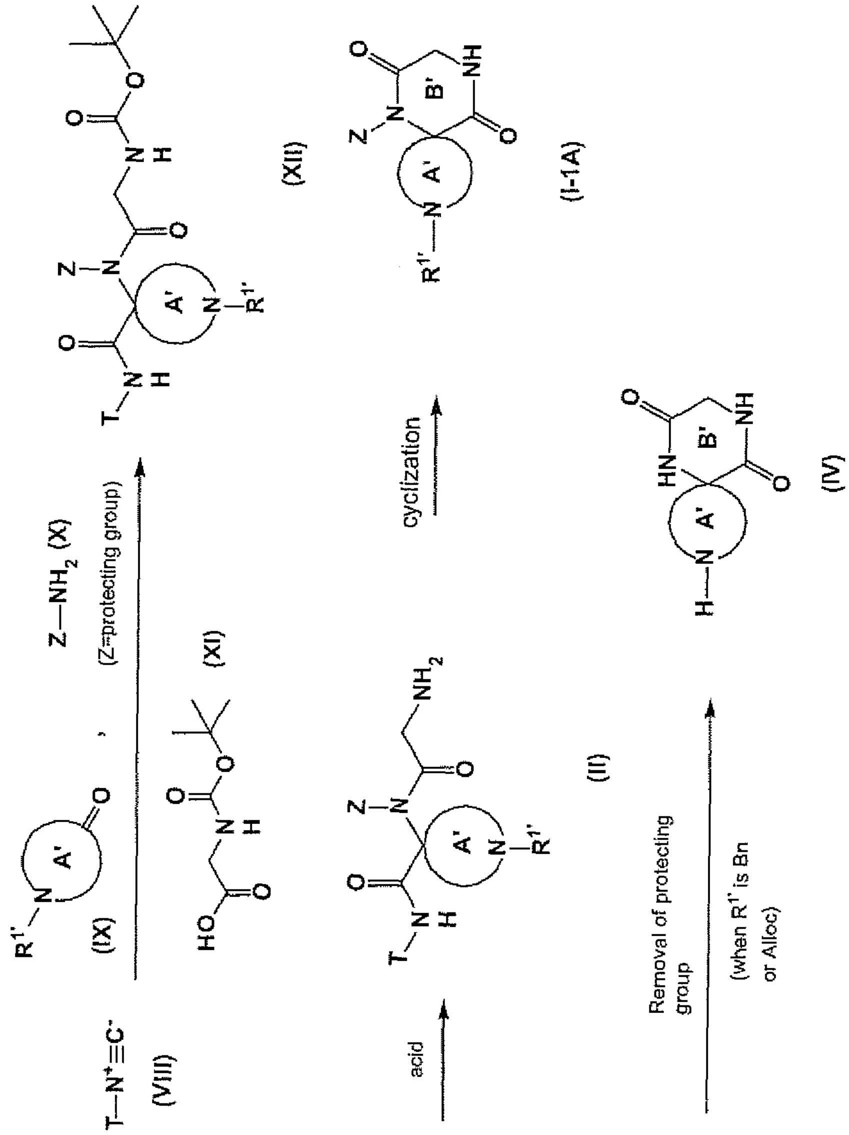


In above Reaction Scheme (1), each reaction may be carried out by known methods. Moreover in Reaction Scheme, the starting materials of the compounds of formula (VIII), formula (IX), formula (X) and formula (XI) may be known *per se* or may be easily prepared by known methods, such as *Comprehensive Organic Transformations: A Guide to Functional Group Preparations,* 2nd Edition (Richard C. Larock, John Wiley & Sons Inc, 1999), in combination.

In each reaction of the specification, it may be used a solid phase reagent which is supported by polymer (for example, polystyrene, polyacrylamide, polypropylene or polyethyleneglycol *etc.).*

In each reaction of the specification, the obtained products may be purified by conventional techniques. For example, the purification may be carried out by distillation at atmospheric or reduced pressure, by high performance liquid chromatography with silica gel or magnesium silicate, by thin layer chromatography, by column chromatography, by washing or by recrystallization. The purification may be done each reaction or after several reactions.

The other starting materials and each reagent in the present invention may be known *per se* or may be prepared by known methods.

Toxicity:

The toxicity of the compounds of the present invention is very low and therefore the compounds may be considered safe for pharmaceutical use.

INDUSTRIAL APPLICABILITY

Application to pharmaceuticals:

The compounds of the present invention of formula (I) antagonize chemokine receptor in animal included human, especially human, so they are used for prevention and/or treatment of various inflammatory diseases (asthma, nephritis, nephropathy, hepatitis, arthritis, chronic rheumatoid arthritis, rhinitis, conjunctivitis, ulcerative colitis and the like), immunologic diseases (autoimmune diseases, transplant rejection, immunosuppression, psoriasis, multiple sclerosis and the like), human immunodeficiency virus infection (acquired immunodeficiency syndrome and the like), allergic diseases (atopic dermatitis, nettle rash, allergic bronchopulmonary aspergillosis, allergic eosinophilic gastroenteritis and the like), ischemia-reperfusion injury, acute respiratory distress syndrome, shock accompanied by bacterial infection, diabetes mellitus, metastasis or the like.

Also, the compound of the present invention represented by formula (I) is useful for prevention and/or treatment of CCR5-related diseases. The CCR5-related diseases include all diseases caused by or related to CCR5 gene.

For the purpose above described, the compounds of formula (1) or salts thereof may be normally administered systemically or locally, usually by oral or parenteral administration.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compounds of the present invention may be administered for example, in the form of solid for oral administration, liquid forms for oral administration, injections, liniments or suppositories for parenteral administration.

Solid forms for oral administration include compressed tablets, pills, capsules, dispersible powders, and granules. Capsules include hard capsules and soft capsules.

In such solid forms, one or more of the active compound(s) may be admixed with vehicles (such as lactose, mannitol, glucose, microcrystalline cellulose or starch), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be coated with two or more films. And further, coating may include containment within capsules of absorbable materials such as gelatin.

Liquid forms for oral administration include pharmaceutically acceptable solutions, suspensions, emulsions, syrups and elixirs. In such forms, one or more of the active compound(s) may be dissolved, suspended or emulsified into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Injections for parenteral administration include all injections. For example, injection into a muscle, injection into a vein, and intravenous drip infusion are included.

Injections for parenteral administration include sterile aqueous, suspensions, emulsions and solid forms which are dissolved or suspended into solvent(s) for injection immediately before use. In injections, one or more of the active compound(s) may be dissolved, suspended or emulsified into solvent(s). The solvents may include distilled water for injection, saline, vegetable oil, propylene glycol, polyethylene glycol, alcohol, e.g. ethanol, or a mixture thereof. Injections may comprise some additives, such as stabilizing agents, solution adjuvants (such as glutamic acid, aspartic acid or POLYSORBATE80 (registered trade mark)), suspending agents, emulsifying agents, soothing agent, buffering agents, preservative. They may be sterilized at a final step, or may be prepared and compensated according to sterile methods. They may also be manufactured in the form of sterile solid forms such as freeze-dried products, which may be dissolved in sterile water or some other sterile diluent(s) for injection immediately before use.

Other forms for parenteral administration include liquids for external use, ointments and endermic liniments, inhalations, sprays, suppositories and pessaries for vaginal administration which comprise one or more of the active compound(s) and may be prepared by methods known *per se.*

Sprays may comprise additional substances other than diluents, such as stabilizing agents, such as sodium sulfate, isotonic buffers, such as sodium chloride, sodium citrate or citric acid. For preparation of such sprays, for example, the method described in the United States Patent No. 2,868,691 or 3,095,355 may be used.

The compound of the present invention represented by formula (I) or a salt thereof may be used together with other drugs, for example, preventive and/or treating agent(s) for HIV infection (particularly an agent for prevention and/or

treatment for AIDS). In that case, the drug as such may be mixed with pharmacologically acceptable excipient, binder, disintegrating agent, lubricant, stabilizer, solubilizer, diluent, *etc.* either separately or simultaneously to make into a pharmaceutical preparation and that can be administered either orally or parenterally as a pharmaceutical composition for prevention and/or treatment of HIV infection.

The compound of the present invention represented by formula (I) or a salt thereof has an infection inhibiting activity to HIV-I which acquired resistance to other agent for prevention and/or treatment for HIV infection (particularly an agent for prevention and/or treatment for AIDS). Therefore, it is also able to be used for HIV-infected patients to whom other agent for prevention and/or treatment for HIV infection is no longer effective. In that case, although the compound of the present invention may be used solely, it may be also used together with an agent for prevention and/or treatment HIV infection where infected HIV-1 strain acquired resistance or with other drugs.

The present invention covers the case where the compound represented by formula (I) or a salt thereof is combined with a drug which does not inhibit the HIV infection whereby preventive and/or treating effect for HIV infection is enhanced as compared with a single preparation.

Examples of other agent for preventive and/or treating HIV infection used for a combination with the compound of the present invention represented by formula (I) or a salt thereof are reverse transcriptase inhibitor, protease inhibitor, chemokine antagonist (such as CCR2 antagonist, CCR3 antagonist, CCR4 antagonist, CCR5 antagonist and CXCR4 antagonist), fusion inhibitor, HIV integrase inhibitor, antibody to surface antigen of HIV-1 and vaccine of HIV-1.

Reverse transcriptase inhibitors are concretely (1) nucleoside/nucleotide reverse transcriptase inhibitors: zidovudine (brand name: Retrovir), didanosine (brand name: Videx), zalcitabine (brand name: HIVID), stavudine (brand name: Zerit), lamivudine (brand name: Epivir), abacavir (brand name: Ziagen), adefovir, adefovir dipivoxil, emtricitabine (brand name: Coviracil) or PMPA (brand name: Tenofovir) *etc.* and (2) nonnucleoside reverse transcriptase inhibitors: nevirapine (brand name: Viramune), delavirdine (brand name: Rescriptor), efavirenz (brand name: Sustiva, Stocklin) or capravirine (AG1549) *etc.*

Protease inhibitors are concretely indinavir (brand name: Crixivan), ritonavir (brand name: Norvir), nelfinavir (brand name: Viracept), saquinavir (brand name: Invirase, Fortovase), amprenavir (brand name: Agenerase), lopinavir (brand name: Kaletra) or tipranavir *etc.*

As chemokine antagonists, internal ligand of chemokine receptor, its derivatives, non-peptide low molecular compound or antibody of chemokine receptor are included.

The examples of internal ligand of chemokine receptor are concretely, MIP-1α, MIP-1β, RANTES, SDF-1α, SDF-1β, MCP-1, MCP-2, MCP-4, Eotaxin and MDC *etc.*

The derivatives of internal ligand are concretely, AOP-RANTES, Met-SDF-1α, Met- SDF-1β *etc.*

Antibodies of chemokine receptor are concretely, Pro-140 *etc.*

CCR2 antagonists are concretely written in specification of WO99/07351, WO99/40913, WO00/46195, WO00/46196, WO00/46197, WO00/46198, WO00/46199, WO00/69432 or WO00/69815 or in *Bioorg. Med. Chem. Lett.,* 10, 1803 (2000) *etc.*

CCR3 antagonists are concretely written in specification of DE19837386, WO99/55324, WO99/55330, WO00/04003, WO00/27800, WO00/27835, WO00/27843, WO00/29377, WO00/31032, WO00/31033, WO00/34278, WO00/35449, WO00/35451, WO00/35452, WO00/35453, WO00/35454, WO00/35876, WO00/35877, WO00/41685, WO00/51607, WO00/51608, WO00/51609, WO00/51610, WO00/53172, WO00/53600, WO00/58305, WO00/59497, WO00/59498, WO00/59502, WO00/59503, WO00/62814, WO00/73327 or WO01/09088 *etc.*

CCR5 antagonists are concretely written in specification of WO99/17773, WO99/32100, WO00/06085, WO00/06146, WO00/10965, WO00/06153, WO00/21916, WO00/37455, EP1013276, WO00/38680, WO00/39125, WO00/40239, WO00/42045, WO00/53175, WO00/42852, WO00/66551, WO00O/66558, WO00/66559, WO00/66141, WO00/68203, JP2000309598, WO00/51607, WO00/51608, WO00/51609, WO00/51610, WO00/56729, WO00/59497, WO00/59498, WO00/59502, WO00/59503, WO00/76933, WO98/25605 or WO99/04794, WO99/38514 or in *Bioorg. Med. Chem. Lett.,* 10, 1803 (2000) *etc.*

CXCR4 antagonists are concretely AMD-3100, T-22, KRH-1120 or the compounds written in specification of WO00/66112 *etc.*

Fusion Inhibitors are concretely, T-20 (Pentafuside) and T-1249 *etc.*

HIV integrase inhibitors are concretely L-chicoric acid, Zintevir, L-870810, PL-2500, Compound B, c-2507 *etc.*

The examples of combination agents written above are intended to illustrate the present invention, but do not limit them.

The typical examples of the usual the dosage level in clinical trials of reverse transcriptase inhibitors or protease inhibitors written below are intended to illustrate the present invention, but do not limit them.

zidovudine: 100 mg capsule, 200 mg per dose, 3 times per day; 300 mg tablet, 300 mg per dose, twice per day;

didanosine: 25-200 mg tablet, 125-200 mg per dose, twice per day;

Table continued

| | |
|---|---|
| zalcitabine: | 0.375-0.75 mg tablet, 0.75 mg per dose, 3 times per day; |
| stavudine: | 15-40 mg capsule, 30-40 mg per dose, twice per day; |
| lamivudine: | 150 mg tablet, 150 mg per dose, twice per day; |
| abacavir: | 300 mg tablet, 300 mg per dose, twice per day; |
| nevirapine: | 200 mg tablet, 200 mg per dose, once per day for 14 days and then twice per day; |
| delavirdine: | 100 mg tablet, 400 mg per dose, 3 times per day; |
| efavirenz: | 50-200 mg capsule, 600 mg per dose, once per day; |
| indinavir: | 200-400 mg capsule, 800 mg per dose, 3 times per day; |
| ritonavir: | 100 mg capsule, 600 mg per dose, twice per day; |
| nelfinavir: | 250 mg tablet, 750 mg per dose, 3 times per day; |
| saquinavir: | 200 mg capsule, 100 or 200 mg per dose, 3 times per day; |
| amprenavir: | 50-150 mg tablet, 100 or 200 mg per dose, twice per day. |

Effect of the invention:

The compounds of the present invention of formula (I), salts thereof and prodrugs thereof are antagonistic against a chemokine receptor (especially CCR5), so that they are used for prevention and/or treatment of various inflammatory diseases (asthma, nephritis, nephropathy, hepatitis, arthritis, chronic rheumatoid arthritis, rhinitis, conjunctivitis, ulcerative colitis and the like), immunologic diseases (autoimmune diseases, transplant rejection, immunosuppression, psoriasis, multiple sclerosis and the like), human immunodeficiency virus infection (acquired immunodeficiency syndrome and the like), allergic diseases (atopic dermatitis, nettle rash, allergic bronchopulmonary aspergillosis, allergic eosinophilic gastroenteritis and the like), ischemia-reperfusion injury, acute respiratory distress syndrome, shock accompanied by bacterial infection, diabetes mellitus, metastasis or the like.

BEST MODE FOR CARRYING OUT THE INVENTION

The following Reference Examples, Examples, Formulation Examples and Biological Examples are intended to illustrate the present invention, but are not limited thereto.
In chromatographic separations and TLC, the solvents in parenthesis show the eluting and developing solvents and the ratios of the solvents used are by volume.
The solvents in parenthesis in NMR show the solvents used for measurement.
Also, when two diastereomer are present, a compound having a smaller Rf value by thin layer silica gel chromatography is represented as a more polar compound, whereas a compound having a larger Rf value is represented as a less polar compound.
The compounds are named using ACD/Name or according to IUPAC nomenclature system.

Example 1

(3 S)-9-benzyl-1-butyl-3-(cyclohexylmethyl)-7-methyl-1,4,9-triazaspiro[5.5]undecane-2,5-dione hydrochloride

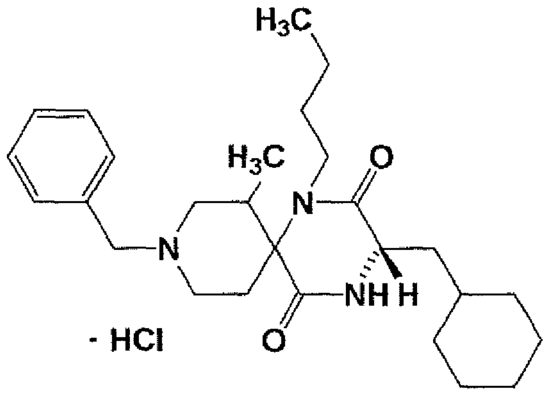

To a methanol solution (20 mL) of 1-benzyl-3-methyl-4-piperidone (203 mg), (2S)-2-(t-butoxycarbonylamino)-3-cyclohexylpropanoic acid (307 mg), and 1.5 equivalents of n-butylamine (110 mg), 1.1 equivalents of 2-morpholinoethylisocyanide (154 mg) was added, followed by stirring at 50°C overnight. After cooling, concentrated hydrochloric acid (1.0 mL) was added thereto, followed by heating and stirring at 60°C for 2 hours. The reaction solution was concentrated, diluted with dichloromethane, and neutralized with a 1N sodium hydrochloride solution. The reaction solution was extracted with dichloromethane, and the organic layer was dried over magnesium sulfate and concentrated. A toluene solution (3 mL) of a 1.25M acetic solution was added thereto, followed by stirring at 100°C overnight. After cooling, the resulting residue was purified by silica gel chromatography (methylene chloride : methanol = 10 : 1). To the resulting residue, a 4N hydrogen chloride ethyl acetate solution was added, followed by concentration to give the compound of the present invention (38 mg) having the following physical data.
TLC: Rf 0.64 (methylene chloride : methanol =10:1);
NMR ($CD_3OD$): δ 7.61-7.48 (m, 5H), 4.38 (d, J = 12.0 Hz, 1H), 4.35 (d, J = 12.0 Hz, 1H), 4.05 (m, 1H), 3.90-3.60 (m, 2H), 3.50-3.10 (m, 3H), 2.70-2.40 (m, 3H), 2.30-2.10 (m, 2H), 1.80-1.10 (m, 4H), 1.00-0.80 (m, 8H).

Examples 1(1) to (7)

By the same procedure as described in Example 1 using 1-benzyl-3-methyl-4-piperidone or the corresponding compound, the following compounds of the present invention were obtained.

Example 1(1)

(3S)-8-benzyl-1-butyl-3-(cyclohexylmetnyl)-1,4,8-triazaspiro[5,5]undecane-2,5-dione hydrochloride
Less polar compound:
TLC: Rf 0.63 (chloroform : methanol = 10 : 1);
NMR ($CD_3OD$):δ 0.82-1.02 (m, 2 H), 0.95 (t, J=7.0 Hz, 3 H), 1.13-1.39 (m, 6 H), 1.42-1.83 (m, 9 H), 1.94-2.28 (m, 4 H), 3.04 (m, 1 H), 3.15 (m, 1 H), 3.33 (m, 1 H), 3.45 (d, J=12.0 Hz, 1 H), 3.57-3.66 (m, 2 H), 3.97 (dd, J=9.0, 4.5 Hz, 1 H), 4.23 (d, J=13.0 Hz, 1 H), 4.55 (d, J=13.0 Hz, 1 H), 7.48-7.55 (m, 5 H).

Example 1(2)

(3S)-8-benzyl-1-butyl-3-(cyclohexylmethyl)-1,4,8-triazaspiro[5.5]undecane-2,5-dione hydrochloride
More polar compound:
TLC: Rf 0.54 (chloroform : methanol = 10 : 1);
NMR ($CD_3OD$): δ 0.84-0.97 (m, 2 H), 0.93 (t, J=7.0 Hz, 3 H), 1.11-1.72 (m, 14 H), 1.81 (m, 1H), 2.00-2.17 (m, 4 H), 3.05 (m, 1 H), 3.17 (m, 1 H), 3.37 (s, 2 H), 3.61 (d, J=11.5 Hz, 1H), 3.70 (m, 1H), 4.10 (dd, J=6.0, 4.5 Hz, 1 H), 4.25 (d, J=13.0 Hz, 1 H), 4.57 (d, J=13.0 Hz, 1H), 7.48-7.57 (m, 5 H).

Example 1(3)

(7S)-2-benzyl-5-butyl-7-(cyclohexylmethyl)-2,5,8-triazaspiro[3.5]nonane-6,9-dione TLC: Rf 0.78(chloroform : methanol = 9 : 1);
NMR ($CD_3OD$): δ 7.36-7.20 (m, 5H), 3.99 (brd, J = 9.0 Hz, 1H), 3.93 (dd, J = 8.1, 5.1 Hz, 1H), 3.83 (m, 2H), 3.74 (s, 2H), 3.68 (brd, J = 9.0 Hz, 1H), 3.65 (brd, J = 9.0 Hz, 1H), 3.57 (brd, J = 9.0 Hz, 1H), 1.79-1.50 (m, 7H), 1.49-1.32 (m,

4H), 1.30-1.10 (m, 4H), 1.01-0.80 (m, 2H), 0.97 (t, J = 7.5 Hz, 3H).

Example 1(4)

(8S)-2-benzyl-6-butyl-8-(cyclohexylmethyl)-2,6,9-triazaspiro[4.5] deeane-7,10-dione hydrochloride
Less polar compound:
TLC: Rf 0.69 (hexane : ethyl acetate =1:1);
NMR ($CD_3OD$): δ 7.62-7.42 (m, 5H), 4.59 (brd, J = 12.6 Hz, 1H), 4.45 (brd, J = 12.6 Hz, 1H), 4.04 (m, 1H), 3.96-3.64 (m, 2H), 3.64-3.00 (m, 4H), 2.61-2.36 (m, 2H), 1.84-1.04 (m, 15H), 1.04-0.80 (m, 5H).

Example 1(5)

(8S)-2-benzyl-6-butyl-8-(cyclohexylmethyl)-2,6,9-triazaspiro[4.5]decane-7,10-dione hydrochloride
More polar compound:
TLC: Rf0.49 (hexane : ethyl acetate =1:1);
NMR ($CD_3OD$): δ 7.62-7.44 (m, 5H), 4.57 (brd, J = 12.9 Hz, 1H), 4.46 (brd, J = 12.9 Hz, 1H), 4.10 (m, 1H), 3.94-3.02 (m, 6H), 2.74-2.30 (m, 2H), 1.81-1.10 (m, 15H), 1.10-0.82 (m, 2H), 0.94 (t, J = 7.2 Hz, 3H).

Example 1(6)

N-[4-(4-{[(3S)-1-butyl-3-(cyclohexylmethyl)-2,5-dioxo-1,4,8-triazaspiro[5.5]undecan-8-yl]methyl}phenoxy)phe nyl]methanesulfonamide hydrochloride
Less polar compound:
TLC: Rf 0.43 (chloroform: methanol = 10 : 1);
NMR ($CD_3OD$): δ 0.83-1.03 (m, 2 H), 0.94 (t, J=7.0 Hz, 3 H), 1.14-1.84 (m, 15 H), 1.96-2.23 (m, 4 H), 2.95 (s, 3 H), 3.00-3.20 (m, 2 H), 3.30 (m, 1 H), 3.50 (d, J=12.0 Hz, 1 H), 3.55-3.67 (m, 2 H), 3.99 (dd, J=9.0, 4.5 Hz, 1 H), 4.22 (d, J=13.5 Hz, 1 H), 4.51 (d, J=13.5 Hz, 1 H), 7.04 (d, J=9.0 Hz, 2 H), 7.05 (d, J=8.5 Hz, 2 H), 7.29 (d, J=9.0 Hz, 2 H), 7.51 (d, J=8.5 Hz, 2 H).

Example 1(7)

N-[4-(4-{[(3S)-1-butyl-3-(cyclohexylmethyl)-2,5-dioxo-1,4,8-triazaspiro[5.5]undecan-8-yl]methyl}phenoxy)phe nyl]methanesulfonamide hydrochloride
More polar compound:
TLC: Rf 0.41 (chloroform : methanol = 10 : 1);
NMR ($CD_3OD$): δ 0.84-0.99 (m, 2 H), 0.93 (t, J=7.5 Hz, 3 H), 1.12-1.74 (m, 14 H), 1.82 (m, 1 H), 1.99-2.17 (m, 4 H), 2.95 (s, 3 H), 3.02-3.21 (m, 2 H), 3.33-3.44 (m, 2 H), 3.60 (dd, J=14.0, 7.0 Hz, 1 H), 3.71 (m, 1H), 4.11 (dd, J=6.0, 4.5 Hz, 1 H), 4.23 (d, J=13.0 Hz, 1 H), 4.53 (d, J=13.0 Hz, 1 H), 7.03 (d, J=9.0 Hz, 2 H), 7.06 (d, J=9.0 Hz, 2 H), 7.29 (d, J=9.0 Hz, 2 H), 7.52 (d, J=9.0 Hz, 2 H).

Biological Example

The fact that the compound of the present invention represented by formula (I) has activity as a CCR5 antagonist was demonstrated, for example, by the following experiment.

The total operation was based on the basic genetic engineering to prepare gene-highly expressing cells, and the ordinary methods were utilized. Also, in the assaying method of the present invention, in order to evaluate the compound of the present invention, assaying accuracy and/or assaying sensitivity was improved as described below. The detailed experimental methods are shown below.

Test methods:

<Pharmaceutical activity of the compounds of the present invention>

(1) Isolation of human CCR5 gene

Human placental cDNA was prepared using Marathon cDNA amplification kit (Clontech). PCR primers hCCR5Xbal-F1:

5'-AGCTAGTCTAGATCCGTTCCCCTACAAGAAACTCTCC-3' (SEQ ID NO:1)

and hCCR5XbaI-R1:

5'-AGCTAGTCTAGAGTGCACAACTCTGACTGGGTCACCA-3' (SEQ ID NO:2)

were designed based on the sequence of GenBank U54994.

Using the human placental cDNA as the template and using Ex Taq (Takara), PCR reaction (2 minutes at 95°C → (30 seconds at 95°C, 45 seconds at 60°C, 1 minute at 72°C) × 35 times) was carried out. The thus amplified PCR product was subjected to a 1% agarose gel electrophoresis, purified using QIAquick Gel Extraction Kit (QUIAGEN) and then digested with a restriction enzyme *Xba*I. The digested fragments were ligated to an expression vector pEF-BOS-bsr using DNA Ligation Kit Ver. 2 (Takara) and transformed into *Escherichia coli* DH5α. By preparing the resulting plasmid pEF-BOS-bsr/hCCR5, its DNA sequence was verified.

(2) Culturing of CHO cell

CHO-dhfr(-) was cultured using Ham's F-12 (containing fetal bovine serum (10%), penicillin (50 U/ml) and streptomycin (50 mg/ml)). Also, the transduced cell was cultured by adding blasticidin (5 mg/ml) to the above medium.

(3) Transduction into CHO cell

The plasmid pEF-BOS-bsr/hCCR5 was transduced into the CHO-dhfr(-) cell using DMREE-C reagent (Gibco BRL). After 48 hours, the medium was replaced with a medium containing 5 mg/ml of blasticidin to carry out the selection, thereby establishing a stably over-expressing cell.

(4) Inhibition test on the binding of RANTES to CCR5 (activity of RANTES to induce transient increase of Ca ion).

The thus established human CCR5 stably over-expressing CHO cell (CCR5/CHO cell) was suspended in Ham's F-12 medium containing FBS (10%) and dispensed in $3.0 \times 10^6$ cells/well portions into a 96 well plate. One day after culturing at 37°C, the culture supernatant was discarded, and Ham's F-12 medium (containing Fura-2AM (5 μM), Probenecid (2.5 mM) and HEPES (20 mM; pH 7.4)) was dispensed in 80 μl/well portions to carry out 1 hour of incubation at 37°C under shaded condition. After washing twice with 1 × Hanks/HEPES (20 mM; pH 7.4) solution, the same solution was dispensed in 100 μl/well portions. Each of the test compounds was added to the thus Fura-2AM-incorporated CCR5/CHO cell, and 3 minutes thereafter, a recombinant human RANTES (PeproTach) diluted with 1 × Hanks/HEPES (20 mM; pH 7.4) solution was added thereto to a final concentration of 10 nM. Transient increase in the intracellular $Ca^{2+}$ concentration induced by the human RANTES was measured using a $Ca^{2+}$ detector for 96 well use (Hamamatsu Photonics), and inhibition ratio (%) of the test compound was calculated by the following calculation formula.

$$\text{Inhibition ratio} = (Ec - Ea)/Ec \times 100$$

Ec: measured value of $Ca^{2+}$ transient increase by RANTES
Ea: measured value of $Ca^{2+}$ transient increase by RANTES when a test compound was added.

As a result, the compounds of the present invention showed an inhibition ratio of 50% or more at 10 μM. For example, the compound of Example 1 showed an $IC_{50}$ value of 0.76μM.

Formulation Example 1

The following components were admixed in a conventional manner, punched out to give 100 tablets each containing 50 mg of active ingredient.

| | |
|---|---|
| * (3S)-9-benzyl-1-butyl-3-(cyclohexylmethyl)-7-methyl-1,4,9-triazaspiro [5.5]undecane-2,5-dione hydrochloride | 5.0 g |
| * calcium carboxymethyl cellulose (disintegrant) | 0.2 g |
| * magnesium stearate(lubricant) | 0.1 g |
| * microcrystalline cellulose | 4.7 g |

Formulation Example 2

The following components were admixed in a conventional technique. The solution was sterilized in a conventional technique, filled in ampoules 5 ml each and freeze-dried over in a conventional technique to give 100 ampoules each containing 20 mg of active ingredient.

| | |
|---|---|
| * (3S)-9-benzyl-1-butyl-3-(cyclohexylmethyl)-7-methyl-1,4,9-triazaspiro[5.5]undecane-2,5-dione hydrochloride | 2.0 g |
| * mannitol | 20 g |
| * distilled water | 500 mL |

## Claims

**1.** A compound represented by formula (I):

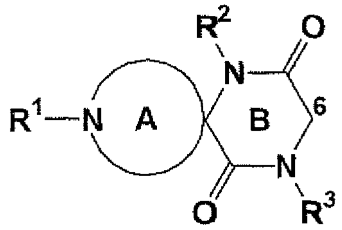

(I)

wherein ring A represents a 3- to 15-membered nitrogen-containing mono-, bi- or tri-cyclic hetero ring which may have a substituent(s);
ring B may have, at the 6-position, an aliphatic hydrocarbon group which may have a substituent(s), a cyclic group which may have a substituent(s), a hydroxyl group which may be protected, a carboxyl group which may be protected, or a carbamoyl group which may be substituted, or

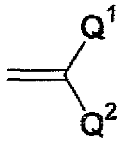

wherein $Q^1$ and $Q^2$ each independently represents hydrogen, an aliphatic hydrocarbon group which may have a substituent(s), a cyclic group which may have a substituent(s), a hydroxyl group which may be protected, a carboxyl group which may be protected, or a carbamoyl group which may be substituted;
$R^1$ represents hydrogen, an aliphatic hydrocarbon group which may have a substituent(s), or a cyclic group which may has a substituent(s);
$R^2$ and $R^3$ each independently represents hydrogen, an aliphatic hydrocarbon group which may have a substituent(s), a cyclic group which may have a substituent(s), a hydroxyl group which may be protected, a carboxyl group which may be protected, or a carbamoyl group which may be substituted, and
wherein, when ring A is

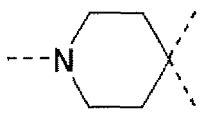 ,

ring A has a substituent(s) other than $R^1$, or
a salt thereof, a solvate thereof, or a prodrug thereof.

2. The compound according to claim 1, wherein ring A is a 4- to 8-membered nitrogen-containing mono-cyclic hetero ring or a 9- to 15-membered nitrogen-containing bi- or tri-cyclic hetero ring which may have a substituent(s), or a salt thereof, a solvate thereof, or a prodrug thereof.

3. The compound according to claim 1, wherein ring A is

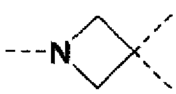 , 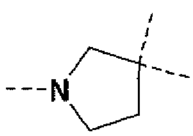 , 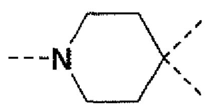 , 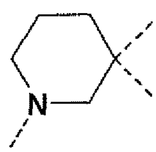 ,

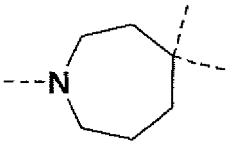, 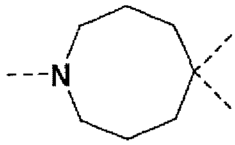, 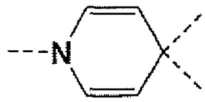, 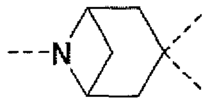,

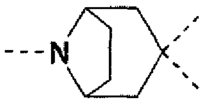, 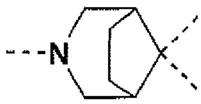, 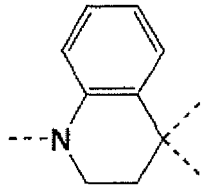, or 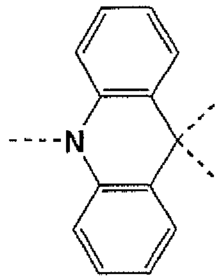

and wherein, when ring A is

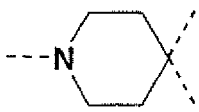,

ring A has a substituent(s) other than $R^1$, or a salt thereof, a solvate thereof, or a prodrug thereof.

4. The compound according to claim 1, wherein $R^1$ is an aliphatic hydrocarbon group which may have a substituent(s), a salt thereof, a solvate thereof, or a prodrug thereof.

5. The compound according to claim 1, wherein ring B is an aliphatic hydrocarbon group which may have a substituent(s), a salt thereof, a solvate thereof, or a prodrug thereof.

6. The compound according to claim 1, wherein $R^3$ is hydrogen, a salt thereof, a solvate thereof, or a prodrug thereof

7. A pharmaceutical composition comprising, as an active ingredient, the compound according to claim 1, a salt thereof, a solvate thereof, or a prodrug thereof.

8. The pharmaceutical composition according to claim 7, which is a chemokine receptor antagonist.

9. The pharmaceutical composition according to claim 8, wherein the chemokine receptor is CCR5.

10. The pharmaceutical composition according to claim 7, which is a preventive and/or therapeutic agent for CCR5-related diseases.

11. The pharmaceutical composition according to claim 7, which is a preventive and/or therapeutic agent for human immunodeficiency virus infection.

12. The pharmaceutical composition according to claim 7, which is a preventive and/or therapeutic agent for acquired immunodeficiency syndrome.

13. The pharmaceutical composition according to claim 7, which is a preventive and/or therapeutic agent for transplanted organ rejection reactions.

14. A medicament which comprises a combination of the compound represented by formula (I) according to claim 1, a salt thereof, a solvate thereof, or a prodrug thereof with one or at least two of agents selected from reverse transcriptase inhibitors, protease inhibitors, CCR2 antagonists, CCR3 antagonists, CCR4 antagonists, CXCR4 antagonists, fusion inhibitors, HIV integrase inhibitors, antibodies against a surface antigen of HIV-1 and vaccines against

HIV-1.

**15.** A method for preventing and/or treating CCR5-related diseases in a mammal, which comprises administering to a mammal an effective amount of the compound represented by formula (I) according to claim 1, a salt thereof, a solvate thereof, or a prodrug thereof

**16.** A method for preventing and/or treating immunodeficiency virus infection in a mammal, which comprises administering to a mammal an effective amount of the compound represented by formula (I) according to claim 1, a salt thereof, a solvate thereof, or a prodrug thereof.

**17.** Use of the compound represented by formula (I) according to claim 1, a salt thereof, a solvate thereof, or a prodrug thereof for the manufacture of a preventive and/or therapeutic agent for CCR5-related diseases.

**18.** Use of the compound represented by formula (I) according to claim 1, a salt thereof, a solvate thereof, or a prodrug thereof for the manufacture of a preventive and/or therapeutic agent for human immunodeficiency virus infection.

**INTERNATIONAL SEARCH REPORT**

| International application No. |
|---|
| PCT/JP2004/005610 |

A. CLASSIFICATION OF SUBJECT MATTER

Int.Cl[7] C07D471/10, A61K31/499, A61P11/06, 13/12, 1/16, 19/02, 11/02, 27/02, 1/04, 37/02, 17/06, 31/18, 37/08, 9/10, 11/00, 35/04, 3/10

According to International Patent Classification (IPC) or to both national classification and IPC

B. FIELDS SEARCHED

Minimum documentation searched (classification system followed by classification symbols)

Int.Cl[7] C07D471/10, A61K31/499, A61P11/06, 13/12, 1/16, 19/02, 11/02, 27/02, 1/04, 37/02, 17/06, 31/18, 37/08, 9/10, 11/00, 35/04, 3/10

Documentation searched other than minimum documentation to the extent that such documents are included in the fields searched

Electronic data base consulted during the international search (name of data base and, where practicable, search terms used)

CASONLINE REGUSTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 01/040227 A1 (Ono Pharmaceutical Co., Ltd.), 07 June, 2001 (07.06.01), & EP 1236726 A1 | 1-10,17 |
| X | JP 2002-348288 A (Ono Pharmaceutical Co., Ltd.), 04 December, 2002 (04.12.02), (Family: none) | 1-13,17,18 |
| X | WO 02/074770 A1 (Ono Pharmaceutical Co., Ltd.), 26 September, 2002 (26.09.02), & US 2004/082584 A1 | 1-10,17 |
| X | WO 02/074769 A1 (Ono Pharmaceutical Co., Ltd.), 26 September, 2002 (26.09.02), & US 2004/106619 A1 | 1-6,11-14, 18 |

[X] Further documents are listed in the continuation of Box C.  [ ] See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 07 July, 2004 (07.07.04) | 03 August, 2004 (03.08.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/005610

C (Continuation). DOCUMENTS CONSIDERED TO BE RELEVANT

| Category* | Citation of document, with indication, where appropriate, of the relevant passages | Relevant to claim No. |
|---|---|---|
| X | Maedaa, K, et al., "Novel Low Molecular Weight Spirodiketopiperazin Derivertives Potently Inhibit R5 HIV-1 Infection through Their Antagonistic Effects on CCR5", Journal of Biological Chemistry, 276(37), pages 35194 to 35200 (2001) | 1-6,11-13, 18 |
| P,X | WO 03/035074 A1 (Ono Pharmaceutical Co., Ltd.), 01 May, 2003 (01.05.03), (Family: none) | 1-7,11-14, 18 |

Form PCT/ISA/210 (continuation of second sheet) (January 2004)

**INTERNATIONAL SEARCH REPORT**

International application No.
PCT/JP2004/005610

**Box No. I Nucleotide and/or amino acid sequence(s) (Continuation of item1.b of the first sheet)**

1. With regard to any nucleotide and/or amino acid sequence disclosed in the international application and necessary to the claimed invention, the international search was carried out on the basis of:

   a. type of material
      - [X] a sequence listing
      - [ ] table(s) related to the sequence listing

   b. format of material
      - [ ] in written format
      - [X] in computer readable form

   c. time of filing/furnishing
      - [ ] contained in the international application as filed
      - [X] filed together with the international application in computer readable form
      - [ ] furnished subsequently to this Authority for the purposes of search

2. [X] In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (January 2004)

**INTERNATIONAL SEARCH REPORT**

International application No.
PCT/JP2004/005610

**Box No. II Observations where certain claims were found unsearchable (Continuation of item 2 of first sheet)**

This international search report has not been established in respect of certain claims under Article 17(2)(a) for the following reasons:

1. [X] Claims Nos.: 15, 16
because they relate to subject matter not required to be searched by this Authority, namely:
Claims 15 and 16 pertain to methods for treatment of the human body by therapy and thus relate to a subject matter which this International Searching Authority is not required to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

**INTERNATIONAL SEARCH REPORT**

International application No.
PCT/JP2004/005610

<Subject of search>

Claim 1 involves a great number of compounds in its scope. However, only small part of the claimed compounds are disclosed in the meaning within PCT Article 5 and thus claim 1 is not fully supported in the meaning within PCT Article 6.

Such being the case, the search was made based on the parts disclosed in the description and supported thereby, i.e., specific EXAMPLES.

Form PCT/ISA/210 (extra sheet) (January 2004)

SEQUENCE LISTING

<110> ONO PHARMACEUTICAL CO., LTD.

<120> The heterocyclic compound containing nitrogen atom and use thereof

<130> ONF-4969PCT

<140> EP 04728443
<141> 2004-03-12

<150> PCT/JP2004/003333
<151> 2004-03-12

<150> JP 2003-116235
<151> 2003-4-21

<160> 2

<170> PatentIn Ver. 2.1

<210> 1
<211> 37
<212> DNA
<213> Artificial Sequence

<220>
<223> Description of Artificial Sequence:Forword primer
hCCR5Xbal

<400> 1
agctagtcta gatccgttcc cctacaagaa actctcc 37

<210> 2
<211> 37
<212> DNA
<213> Artificial Sequence

<220>
<223> Description of Artificial Sequence:Revese primer
hCCR5Xbal

<400> 2
agctagtcta gagtgcacaa ctctgactgg gtcacca 37